# EUROPÄISCHE PATENTSCHRIFT

(11) **EP 4 750 414 B1**
(45) Veröffentlichungstag und Bekanntmachung des Hinweises auf die Patenterteilung: **04.11.2026**
(21) Anmeldenummer: 24748581.6
(22) Anmeldetag: 23.07.2024
(51) Int. Cl.: A61B 90/98, H01R 13/00, A61B 17/00, A61B 90/00

(54) **CHIRURGISCHE VORRICHTUNG MIT RFID-CHIP UND RFID-ANTENNE**
SURGICAL DEVICE WITH RFID CHIP AND RFID ANTENNA
DISPOSITIF CHIRURGICAL AVEC PUCE RFID ET ANTENNE RFID

(30) Priorität: 26.07.2023 DE 102023119808
(43) Veröffentlichungstag der Anmeldung: 03.06.2026
(73) Patentinhaber: Aesculap AG, 78532 Tuttlingen (DE)
(72) Erfinder: BUERK, Andre, 78056 Villingen-Schwenningen (DE); HOEGERLE, Roland-Alois, 78532 Tuttlingen (DE); LENZENHUBER, Frederick, 78532 Tuttlingen (DE); KAHLER, Thomas-Erwin, 78606 Seitingen-Oberflacht (DE); MACHILL, Martin, 78604 Rietheim-Weilheim (DE)
(74) Vertreter: Winter, Brandl - Partnerschaft mbB
(86) Internationale Anmeldenummer: PCT/EP2024/070837
(87) Internationale Veröffentlichungsnummer: WO 2025/021796

(56) Entgegenhaltungen:
- WO-A1-2021/069662
- DE-A1- 102011 050 192
- US-A1- 2015 048 139

## Beschreibung

### Technisches Gebiet

Die vorliegende Offenbarung betrifft eine chirurgische Vorrichtung, die ein chirurgisches Anwendungsteil, das einen Elektromotor mit Motorwicklungen aufweist, und ein elektrisches Versorgungskabel, das eine einer Anzahl der Motorwicklungen entsprechende Anzahl von Leitungen aufweist und mit dem Anwendungsteil koppelbar ist, um dem Elektromotor Energie zuzuführen, aufweist.

### Stand der Technik

Eine derartige chirurgische Vorrichtung ist aus der DE 10 2011 050 192 A1 bekannt. Darin ist ein chirurgisches Kupplungssystem mit zwei Kupplungsvorrichtungen, welche jeweils mechanisch und elektrisch miteinander in Eingriff bringbare Kupplungskontakte aufweisen, beschrieben. Dabei ist eine erste Kupplungsvorrichtung einem chirurgischen Anwendungsteil und eine zweite Kupplungsvorrichtung einem elektrischen Versorgungskabel zugeordnet. Die erste und die zweite Kupplungsvorrichtung sind derart ausgebildet, dass sie zwei Schaltzustände, nämlich einen AUS-Zustand und einen AN-Zustand, einnehmen können. In dem AUS-Zustand kann anhand eines Kodier-/Erkennungswiderstands bestimmt werden, welcher Anwendungsteil-Typ mit dem elektrischen Versorgungskabel gekoppelt ist. In dem AN-Zustand kann der Elektromotor in dem Anwendungsteil dann anhand dieser Information durch eine mit einem anderen Ende des Versorgungskabels verbundene Steuereinheit entsprechend betrieben bzw. angesteuert werden. Nachteilig dabei ist, dass die Anzahl an verwendbaren Widerständen begrenzt ist, so dass nur ein allgemeiner Typ des Anwendungsteils, z.B. Fräser, Säge usw., bestimmbar ist. Demzufolge können keine weitergehenden Daten bzw. Informationen durch die Steuereinheit abgefragt werden.

Aus diesem Grund offenbart die WO 2021 / 069 662 A1 eine chirurgische Vorrichtung mit einer integrierten RFID-Ausleseantenne als eine Weiterbildung der obigen chirurgischen Vorrichtung.

Eine weitere derartige chirurgische Vorrichtung ist außerdem aus US 2015 / 0 048 139 A1 bekannt.

Es ist Aufgabe der vorliegenden Offenbarung, eine weiterentwickelte chirurgische Vorrichtung bereitzustellen.

Diese Aufgabe wird durch die chirurgische Vorrichtungen mit den Merkmalen gemäß den unabhängigen Ansprüchen gelöst. Vorteilhafte Ausführungsformen sind Gegenstand der abhängigen Ansprüche und/oder sind in der Beschreibung und/oder den Figuren offenbart.

Eine offenbarungsgemäße chirurgische Vorrichtung weist ein chirurgisches Anwendungsteil, das einen Elektromotor mit Motorwicklungen und an seinem einen Ende eine erste Kupplungsvorrichtung aufweist, auf. Der Elektromotor kann als Wechselstrommotor oder bevorzugter als Drehstrommotor mit drei oder einem Vielfachen von drei Motorwicklungen ausgebildet sein.

Um dem Anwendungsteil, d.h. dem Elektromotor, Energie zuzuführen, weist die chirurgische Vorrichtung ein elektrisches Versorgungskabel mit einer einer Anzahl der Motorwicklungen entsprechenden Anzahl von Leitungen auf. Folglich kann das elektrische Versorgungskabel bei Einsatz des Wechselstrommotors zwei Leitungen aufweisen. Bei Einsatz des Drehstrommotors kann das elektrische Versorgungskabel drei Leitungen aufweisen, um die drei oder die einem Vielfachen von drei, z.B. sechs, Motorwicklungen mit Energie zu versorgen. Das elektrische Versorgungskabel weist an seinem einen Ende eine zweite Kupplungsvorrichtung, die zum Koppeln mit der ersten Kupplungsvorrichtung eingerichtet ist, auf. An seinem zweiten Ende ist das elektrische Versorgungskabel mit einer Steuereinheit zum Steuern des Elektromotors verbunden oder, z.B. mittels einer dafür vorgesehenen Steuereinheit-Kupplungsvorrichtung, verbindbar.

Die erste und die zweite Kupplungsvorrichtung sind so eingerichtet, dass sie in einen Trennzustand oder in einen AUS-Zustand oder in einen AN-Zustand bringbar sind. In dem Trennzustand sind die erste und die zweite Kupplungsvorrichtung sowohl mechanisch als auch elektrisch vollständig außer Eingriff. In dem AUS-Zustand sind die erste und zweite Kupplungsvorrichtung zumindest teilweise mechanisch in Eingriff und zumindest einige der Kupplungskontakte sind elektrisch und mechanisch in Eingriff. In dem AN-Zustand sind die erste und die zweite Kupplungsvorrichtung vollständig mechanisch in Eingriff und Kupplungskontakte, die zum Versorgen des Elektromotors mit Energie dienen, sind sowohl elektrisch als auch mechanisch in Eingriff. Diese Art der ersten und der zweiten Kupplungsvorrichtung ist bereits aus der DE 10 2011 050 192 A1 bekannt.

Gemäß der vorliegenden Offenbarung sind in dem AUS-Zustand erste Kupplungskontakte der ersten und der zweiten Kupplungsvorrichtung elektrisch in Eingriff. Der erste Kupplungskontakt in der ersten Kupplungsvorrichtung ist mit einer ersten Motorwicklung verbunden und der erste Kupplungskontakt in der zweiten Kupplungsvorrichtung ist mit einer ersten Leitung in dem elektrischen Versorgungskabel verbunden. Ferner sind zweite Kupplungskontakte der ersten und der zweiten Kupplungsvorrichtung elektrisch außer Eingriff. Der zweite Kupplungskontakt in der ersten Kupplungsvorrichtung ist mit einer zweiten Motorwicklung verbunden und der zweite Kupplungskontakt der zweiten Kupplungsvorrichtung ist mit einer zweiten Leitung in dem elektrischen Versorgungskabel verbunden. Demzufolge ist in dem AUS-Zustand eine Energieversorgung zwischen der zweiten Leitung und der zweiten Motorwicklung unterbrochen, so dass der Elektromotor nicht in Betrieb ist.

Gemäß der vorliegenden Offenbarung sind in dem AN-Zustand die ersten Kupplungskontakte der ersten und der zweiten Kupplungsvorrichtung und die zweiten Kupplungskontakte der ersten und der zweiten Kupplungsvorrichtung elektrisch und mechanisch in Eingriff. Demzufolge wird in dem AN-Zustand die zweite Motorwicklung mit Energie über die zweite Leitung versorgt, um den Elektromotor anzutreiben.

Die chirurgische Vorrichtung weist einen in der ersten Kupplungsvorrichtung angeordneten RFID-Chip auf. Der RFID-Chip kann bevorzugt als passiver Glass-Tag ausgebildet sein. In dem RFID-Chip kann eine Vielzahl von Daten bzw. Informationen gespeichert sein. Die Daten können beispielsweise eine Seriennummer, ein Wartungsintervall, eine Art der durchgeführten Wartungen, ein Herstellungsdatum, eine Leistung, einen Anwendungszähler und/oder eine Anwendungsdauer umfassen. Demzufolge wird im Service- bzw. Fehlerfall eine hohe Transparenz ermöglicht.

Um die in dem RFID-Chip gespeicherten Daten auszulesen, weist die chirurgische Vorrichtung eine in der ersten Kupplungsvorrichtung angeordnete und mit Energie versorgbare RFID-Antenne auf. Die RFID-Antenne ist benachbart, d.h. in unmittelbarer Nähe, zu dem RFID-Chip angeordnet und ausgebildet, den RFID-Chip anzuregen, um Daten zwischen dem RFID-Chip und der Steuereinheit bevorzugt bidirektional zu übertragen. Demzufolge können die in dem RFID-Chip gespeicherten Daten durch die Steuereinheit gelesen werden und eine Verwendung des Anwendungsteils kann durch die Steuereinheit beispielsweise nur freigegeben werden, wenn die gespeicherten Daten vorher festgelegten Bedingungen genügen. Dadurch kann zum Beispiel eine Verwendung eines nicht gewarteten Anwendungsteils zuverlässig vermieden werden. Bevorzugt kann die Steuereinheit auch Daten in den RFID-Chip schreiben, so dass die Daten nach Verwendung des Anwendungsteils aktualisiert sind. Die RFID-Antenne kann beispielsweise als Draht, insbesondere Kupferdraht, gedruckte Schaltkreisplatine mit Leiterbahnen, flexible gedruckte Schalkreisplatine mit Leiterbahnen, Kunststoff mit aktivierten Leiterbahnen, als Zwei-Komponenten-Spritzguss mit Leiterbahnen oder als Schlitzantenne ausgebildet sein.

Offenbarungsgemäß weisen die erste und die zweite Kupplungsvorrichtung jeweils einen weiteren elektrischen Kupplungskontakt, der in der ersten Kupplungsvorrichtung über die RFID-Antenne mit dem ersten Kupplungskontakt parallel verbunden ist und in der zweiten Kupplungsvorrichtung mit dem zweiten Kupplungskontakt parallel verbunden ist, auf. Die weiteren Kupplungskontakte der ersten und der zweiten Kupplungsvorrichtung sind so eingerichtet, dass sie in dem AUS-Zustand elektrisch in Eingriff sind und in dem AN-Zustand elektrisch außer Eingriff sind. Demnach wird in dem AUS-Zustand eine Versorgung der RFID-Antenne mit Energie erreicht. Die Energie zum Betreiben der RFID-Antenne wird dabei über die erste Leitung, den ersten Kupplungskontakt in der zweiten Kupplungsvorrichtung, den ersten Kupplungskontakt in der ersten Kupplungsvorrichtung, die RFID-Antenne, den weiteren Kupplungskontakt in der ersten Kupplungsvorrichtung, den weiteren Kupplungskontakt in der zweiten Kupplungsvorrichtung und die zweite Leitung bereitgestellt. Da die zweiten Kupplungskontakte in der ersten Kupplungsvorrichtung und der zweiten Kupplungsvorrichtung in dem AUS-Zustand elektrisch außer Eingriff sind, wird ein Betrieb des Elektromotors unterbunden. Zudem ist keine Buchse mit Kontaktbrücke und federndem Element zum Rückstellen der Kontaktbrücke, wie sie in der WO 2021 / 069 662 A1 verwendet wird, erforderlich. Darüber hinaus ist es auch nicht erforderlich, dass das elektrische Versorgungskabel mehr als die der Anzahl der Motorwicklungen entsprechende Anzahl von Leitungen aufweist.

Bevorzugt können die erste und die zweite Kupplungsvorrichtung jeweils dritte Kupplungskontakte aufweisen, die in dem AUS-Zustand und in dem AN-Zustand elektrisch in Eingriff sind. Demzufolge kann in dem AUS-Zustand eine leitende Verbindung über die erste Leitung, den ersten Kupplungskontakt in der zweiten Kupplungsvorrichtung, den ersten Kupplungskontakt in der ersten Kupplungsvorrichtung, die erste Motorwicklung, die dritte Motorwicklung, den dritten Kupplungskontakt in der ersten Kupplungsvorrichtung, den dritten Kupplungskontakt in der zweiten Kupplungsvorrichtung und die dritte Leitung erreicht werden. Demzufolge können die erste und die dritte Motorwicklung des Elektromotors, insbesondere deren Serienwiderstand, zum Bestimmen eines Typs des Elektromotors in dem Anwendungsteil durch die Steuereinheit gemessen werden. Dies kann zeitgleich zu einem Empfangen/Schreiben von Daten aus/in den RFID-Chip erfolgen.

Alternativ können die erste und die zweite Kupplungsvorrichtung dritte Kupplungskontakte aufweisen, die in dem AUS-Zustand elektrisch außer Eingriff und in dem AN-Zustand elektrisch in Eingriff sind. Das heißt, die dritten Kupplungskontakte sind zumindest in dem AN-Zustand elektrisch in Eingriff, um eine Versorgung des Elektromotors mit Energie zu ermöglichen.

Des Weiteren können die Kupplungskontakte in der ersten Kupplungsvorrichtung als Stifte und in der zweiten Kupplungsvorrichtung als Buchsen ausgebildet sein. Der Stift des weiteren Kupplungskontakts kann länger als der Stift des zweiten Kupplungskontakts ausgebildet sein. Demzufolge wird ein elektrisch leitender Kontakt der weiteren Kupplungskontakte in dem AUS-Zustand erreicht, während die zweiten Kupplungskontakte elektrisch und auch mechanisch außer Eingriff sind. Der Stift des weiteren Kupplungskontakts kann einen Isolierabschnitt aufweisen, der so ausgebildet ist, dass die weiteren Kupplungskontakte in dem AN-Zustand elektrisch außer Eingriff sind. Insbesondere kann ein Schaftabschnitt des als Stift ausgebildeten Kupplungskontakts mit einer Isolierschicht abgedeckt bzw. beschichtet sein, um den Isolierabschnitt zu bilden. Die Isolierschicht ist bevorzugt so gebildet, dass sie zu einem leitenden Abschnitt an der Spitze des Stifts im Wesentlichen plan ist. Alternativ oder zusätzlich kann die Buchse des weiteren Kupplungskontakts einen Isolierabschnitt aufweisen, der so ausgebildet ist, dass die dritten Kupplungskontakte in dem AN-Zustand elektrisch außer Eingriff sind. Insbesondere kann ein Bodenabschnitt der Buchse mit einer Isolierschicht abgedeckt sein, um den Isolierabschnitt zu bilden. Die Isolierschicht ist bevorzugt im Wesentlichen plan zu einem leitenden Abschnitt an einem Eingangsabschnitt der Buchse gebildet. Auf diese Weise wird eine elektrisch leitende Verbindung der weiteren Kupplungskontakte in dem AUS-Zustand und eine elektrisch nicht-leitende Verbindung in dem AN-Zustand erreicht.

Mit Vorteil können der weitere Kupplungskontakt und der erste Kupplungskontakt in der ersten Kupplungsvorrichtung über eine Drahtbrücke parallel miteinander verbunden sein. Demzufolge kann eine einfache Verbindung zwischen den beiden Kupplungskontakten erreicht werden. Die Drahtbrücke kann ggf. auch für vorhandene vierpolige erste Kupplungsvorrichtungen nachgerüstet werden.

Weiterhin kann die chirurgische Vorrichtung einen Erkennungswiderstand aufweisen, der mit der RFID-Antenne wenigstens in dem AUS-Zustand in Serie verbunden ist. Der Erkennungswiderstand kann dabei in der ersten Kupplungsvorrichtung oder in der zweiten Kupplungsvorrichtung angeordnet sein. Bevorzugt kann der Erkennungswiderstand in der ersten Kupplungsvorrichtung angeordnet sein und einen dem Anwendungsteil oder dem Elektromotor entsprechenden Widerstandswert aufweisen. Der Erkennungswiderstand kann beispielsweise einen Widerstandswert von 10 Ohm aufweisen. Demzufolge kann über den oben beschriebenen Pfad zum Versorgen der RFID-Antenne ein Serienwiderstand zwischen dem Erkennungswiderstand und dem Widerstand der RFID-Antenne vor und/oder während einer Anregung der RFID-Antenne durch die Steuereinheit erfasst werden, um das ordnungsgemäße Vorliegen des AUS-Zustands zu bestimmen. Dieses Vorgehen ist vor allem dann vorteilhaft, wenn die dritten Kupplungskontakte der ersten Kupplungsvorrichtung und der zweiten Kupplungsvorrichtung in dem AUS-Zustand elektrisch außer Eingriff sind und keine Messung des Serienwiderstands von Motorwicklungen möglich ist. Da der Serienwiderstand der Motorwicklungen um Bereich weniger Ohm liegt, ist das Vorsehen des Erkennungswiderstands aber auch dann vorteilhaft, wenn der Serienwiderstand der Motorwicklungen über die dritten Kupplungskontakte messbar ist.

Außerdem kann die erste Kupplungsvorrichtung einen Kupplungszapfen aufweisen. Der RFID-Chip kann radial außenliegend in dem Kupplungszapfen angeordnet sein. Demzufolge kann der RFID-Chip auch von außerhalb der Kupplungsvorrichtung durch eine externe Vorrichtung, die eine RFID-Antenne aufweist und an den Kupplungszapfen im Bereich des RFID-Chips gehalten wird, ausgelesen/beschrieben werden. Folglich ist keine Verbindung mit der zweiten Kupplungsvorrichtung des elektrischen Versorgungskabels zum Auslesen/Beschreiben des RFID-Chips erforderlich. Der Kupplungszapfen kann radial innenliegend wiederum eine Kupplungshülse bilden, in die ein Kupplungszapfen der zweiten Kupplungsvorrichtung einfügbar ist. Die Kupplungshülse kann mit einem geraden Abschnitt geformt sein, um eine Wandstärke zu vergrößern und einen Aufnahmeraum zu definieren. Der Kupplungszapfen und die Kupplungshülse können aus einem äußeren und einem inneren Element gebildet sein. Zwischen dem äußeren und dem inneren Element kann ein Stirnseitenelement angeordnet sein, um den Zwischenraum zu verschließen. Demnach kann der RFID-Chip auf einfache Weise in die erste Kupplungsvorrichtung integriert werden.

Bevorzugt kann die RFID-Antenne in dem Kupplungszapfen der ersten Kupplungsvorrichtung radial innenliegend zu dem RFID-Chip angeordnet sein. Falls in dem Kupplungszapfen eine Kupplungshülse gebildet ist, kann die RFID-Antenne zur Kupplungshülse hin, bevorzugt entlang des geraden Abschnitts der Kupplungshülse, angeordnet sein. Auf diese Weise ist die RFID-Antenne nahe an dem RFID-Chip angeordnet, so dass ein zuverlässiges Auslesen/Beschreiben des RFID-Chips erreicht wird.

Dabei kann die RFID-Antenne als Schlitzantenne ausgebildet sein. Demzufolge weist die RFID-Antenne eine hohe Robustheit auf und kann auf einfache Weise in die erste Kupplungsvorrichtung, bevorzugt entlang des geraden Abschnitts, integriert werden.

Mit Vorteil kann eine elektrische Schaltung vorgesehen sein, die so konfiguriert ist, dass sie die RFID-Antenne abstimmt. Die elektrische Schaltung kann dabei in der zweiten Kupplungsvorrichtung angeordnet sein. Demnach ist nur die RFID-Antenne im Bereich der zweiten Kupplungsvorrichtung abzustimmen. Alternativ kann die elektrische Schaltung in der Steuereinheit angeordnet sein. Folglich sind auch die Leitungen zum Versorgen der RFID-Antenne mit Energie abzustimmen. Durch die Abstimmung der RFID-Antenne mittels der elektrischen Schaltung wird eine zuverlässige Datenübertragung zwischen dem RFID-Chip und der Steuereinheit über die RFID-Antenne erreicht.

In der zweiten Kupplungsvorrichtung kann eine elektrische Schaltung angeordnet sein, die so konfiguriert ist, dass sie die von der RFID-Antenne empfangenen Daten verarbeitet und an die Steuereinheit überträgt und/oder die von der Steuereinheit empfangenen Daten verarbeitet und in den RFID-Chip schreibt. Die elektrische Schaltung kann dazu einen Mikrocomputer bzw. Mikrocontroller aufweisen. Die elektrische Schaltung kann die von der RFID-Antenne oder der Steuereinheit empfangenen Daten insbesondere von einer Form in eine andere Form und/oder wieder zurück wandeln. Bevorzugt kann die elektrische Schaltung die von der RFID-Antenne empfangenen Daten mittels der One-Wire-Technik an die Steuereinheit übertragen und/oder von dieser empfangen. Demzufolge wird eine zuverlässige bidirektionale Datenübertragung zwischen der elektrischen Schaltung und der Steuereinheit auch bei großen Kabellängen erreicht. Es ist anzumerken, dass die beiden beschriebenen elektrischen Schaltungen in einer elektrischen Schaltung integriert sein können.

Eine offenbarungsgemäße chirurgische Vorrichtung weist ein chirurgisches Anwendungsteil, das einen Elektromotor mit Motorwicklungen und an seinem einen Ende eine erste Kupplungsvorrichtung aufweist, auf. Der Elektromotor kann als Wechselstrommotor oder bevorzugter als Drehstrommotor mit drei oder einem Vielfachen von drei Motorwicklungen ausgebildet sein.

Um dem Anwendungsteil, d.h. dem Elektromotor, Energie zuzuführen, weist die chirurgische Vorrichtung ein elektrisches Versorgungskabel mit einer einer Anzahl der Motorwicklungen entsprechenden Anzahl von Leitungen auf. Folglich kann das elektrische Versorgungskabel bei Einsatz des Wechselstrommotors zwei Leitungen aufweisen. Bei Einsatz des Drehstrommotors kann das elektrische Versorgungskabel drei Leitungen aufweisen, um die drei oder die einem Vielfachen von drei, z.B. sechs, Motorwicklungen mit Energie zu versorgen. Das elektrische Versorgungskabel weist an seinem einen Ende eine zweite Kupplungsvorrichtung, die zum Koppeln mit der ersten Kupplungsvorrichtung eingerichtet ist, auf. An seinem zweiten Ende ist das elektrische Versorgungskabel mit einer Steuereinheit zum Steuern des Elektromotors verbunden oder, z.B. mittels einer dafür vorgesehene Steuereinheit-Kupplungsvorrichtung, verbindbar.

Die erste und die zweite Kupplungsvorrichtung sind so eingerichtet, dass sie in einen Trennzustand oder in einen AUS-Zustand oder in einen AN-Zustand bringbar sind. In dem Trennzustand sind die erste und die zweite Kupplungsvorrichtung sowohl mechanisch als auch elektrisch vollständig außer Eingriff. In dem AUS-Zustand sind die erste und zweite Kupplungsvorrichtung zumindest teilweise mechanisch in Eingriff und zumindest einige der Kupplungskontakte sind elektrisch und mechanisch in Eingriff. In dem AN-Zustand sind die erste und die zweite Kupplungsvorrichtung vollständig mechanisch in Eingriff und Kupplungskontakte, die zum Versorgen des Elektromotors mit Energie dienen, sind sowohl elektrisch als auch mechanisch in Eingriff. Diese Art der ersten und der zweiten Kupplungsvorrichtung ist bereits aus der DE 10 2011 050 192 A1 bekannt.

Gemäß der vorliegenden Offenbarung sind in dem AUS-Zustand erste Kupplungskontakte der ersten und der zweiten Kupplungsvorrichtung elektrisch in Eingriff. Der erste Kupplungskontakt in der ersten Kupplungsvorrichtung ist mit einer ersten Motorwicklung verbunden und der erste Kupplungskontakt in der zweiten Kupplungsvorrichtung ist mit einer ersten Leitung in dem elektrischen Versorgungskabel verbunden. Ferner sind zweite Kupplungskontakte der ersten und der zweiten Kupplungsvorrichtung elektrisch außer Eingriff. Der zweite Kupplungskontakt in der ersten Kupplungsvorrichtung ist mit einer zweiten Motorwicklung verbunden und der zweite Kupplungskontakt der zweiten Kupplungsvorrichtung ist mit einer zweiten Leitung in dem elektrischen Versorgungskabel verbunden. Demzufolge ist in dem AUS-Zustand eine Energieversorgung zwischen der zweiten Leitung und der zweiten Motorwicklung unterbrochen, so dass der Elektromotor nicht in Betrieb ist.

Gemäß der vorliegenden Offenbarung sind in dem AN-Zustand die ersten Kupplungskontakte der ersten und der zweiten Kupplungsvorrichtung und die zweiten Kupplungskontakte der ersten und der zweiten Kupplungsvorrichtung elektrisch und mechanisch in Eingriff. Demzufolge wird in dem AN-Zustand die zweite Motorwicklung mit Energie über die zweite Leitung versorgt, um den Elektromotor anzutreiben.

Die chirurgische Vorrichtung weist einen in der ersten Kupplungsvorrichtung angeordneten RFID-Chip auf. Der RFID-Chip kann bevorzugt als passiver Glass-Tag ausgebildet sein. In dem RFID-Chip kann eine Vielzahl von Daten bzw. Informationen gespeichert sein. Die Daten können beispielsweise eine Seriennummer, ein Wartungsintervall, eine Art der durchgeführten Wartungen, ein Herstellungsdatum, eine Leistung, einen Anwendungszähler und/oder eine Anwendungsdauer umfassen. Demzufolge wird im Service- bzw. Fehlerfall eine hohe Transparenz ermöglicht.

Um die in dem RFID-Chip gespeicherten Daten auszulesen, weist die chirurgische Vorrichtung eine in der zweiten Kupplungsvorrichtung angeordnete und mit Energie versorgbare RFID-Antenne auf. Die RFID-Antenne ist zumindest in dem AUS-Zustand benachbart, d.h. in unmittelbarer Nähe, zu dem RFID-Chip angeordnet und ausgebildet, den RFID-Chip anzuregen, um Daten zwischen dem RFID-Chip und der Steuereinheit bevorzugt bidirektional zu übertragen. Demzufolge können die in dem RFID-Chip gespeicherten Daten durch die Steuereinheit gelesen werden und eine Verwendung des Anwendungsteils kann durch die Steuereinheit beispielsweise nur freigegeben werden, wenn die gespeicherten Daten vorher festgelegten Bedingungen genügen. Dadurch kann zum Beispiel eine Verwendung eines nicht gewarteten Anwendungsteils zuverlässig vermieden werden. Bevorzugt kann die Steuereinheit auch Daten in den RFID-Chip schreiben, so dass die Daten nach Verwendung des Anwendungsteils aktualisiert sind. Die RFID-Antenne kann beispielsweise als Draht, insbesondere Kupferdraht, gedruckte Schaltkreisplatine mit Leiterbahnen, flexible gedruckte Schalkreisplatine mit Leiterbahnen, Kunststoff mit aktivierten Leiterbahnen, als Zwei-Komponenten-Spritzguss mit Leiterbahnen oder als Schlitzantenne ausgebildet sein.

Offenbarungsgemäß weisen die erste und die zweite Kupplungsvorrichtung jeweils einen weiteren elektrischen Kupplungskontakt, der in der ersten Kupplungsvorrichtung mit dem ersten Kupplungskontakt parallel verbunden ist und in der zweiten Kupplungsvorrichtung über die RFID-Antenne mit dem zweiten Kupplungskontakt parallel verbunden ist, auf. Die weiteren Kupplungskontakte der ersten und der zweiten Kupplungsvorrichtung sind so eingerichtet, dass sie in dem AUS-Zustand elektrisch in Eingriff sind und in dem AN-Zustand elektrisch außer Eingriff sind. Demnach wird in dem AUS-Zustand eine Versorgung der RFID-Antenne mit Energie erreicht. Die Energie zum Betreiben der RFID-Antenne wird dabei über die erste Leitung, den ersten Kupplungskontakt in der zweiten Kupplungsvorrichtung, den ersten Kupplungskontakt in der ersten Kupplungsvorrichtung, den weiteren Kupplungskontakt in der ersten Kupplungsvorrichtung, den weiteren Kupplungskontakt in der zweiten Kupplungsvorrichtung, die RFID-Antenne und die zweite Leitung bereitgestellt. Da die zweiten Kupplungskontakte in der ersten Kupplungsvorrichtung und der zweiten Kupplungsvorrichtung in dem AUS-Zustand elektrisch außer Eingriff sind, wird ein Betrieb des Elektromotors unterbunden. Zudem ist keine Buchse mit Kontaktbrücke und federndem Element zum Rückstellen der Kontaktbrücke, wie sie in der WO 2021 / 069 662 A1 verwendet wird, erforderlich. Darüber hinaus ist es auch nicht erforderlich, dass das elektrische Versorgungskabel mehr als die der Anzahl der Motorwicklungen entsprechende Anzahl von Leitungen aufweist.

Bevorzugt können die erste und die zweite Kupplungsvorrichtung jeweils dritte Kupplungskontakte aufweisen, die in dem AUS-Zustand und in dem AN-Zustand elektrisch in Eingriff sind. Demzufolge kann in dem AUS-Zustand eine leitende Verbindung über die erste Leitung, den ersten Kupplungskontakt in der zweiten Kupplungsvorrichtung, den ersten Kupplungskontakt in der ersten Kupplungsvorrichtung, die erste Motorwicklung, die dritte Motorwicklung, den dritten Kupplungskontakt in der ersten Kupplungsvorrichtung, den dritten Kupplungskontakt in der zweiten Kupplungsvorrichtung und die dritte Leitung erreicht werden. Demzufolge können die erste und die dritte Motorwicklung des Elektromotors, insbesondere deren Serienwiderstand, zum Bestimmen eines Typs des Elektromotors in dem Anwendungsteil durch die Steuereinheit gemessen werden. Dies kann zeitgleich zu einem Empfangen/Schreiben von Daten aus/in den RFID-Chip erfolgen.

Alternativ können die erste und die zweite Kupplungsvorrichtung dritte Kupplungskontakte aufweisen, die in dem AUS-Zustand elektrisch außer Eingriff und in dem AN-Zustand elektrisch in Eingriff sind. Das heißt, die dritten Kupplungskontakte sind zumindest in dem AN-Zustand elektrisch in Eingriff, um eine Versorgung des Elektromotors mit Energie zu ermöglichen.

Des Weiteren können die Kupplungskontakte in der ersten Kupplungsvorrichtung als Stifte und in der zweiten Kupplungsvorrichtung als Buchsen ausgebildet sein. Der Stift des weiteren Kupplungskontakts kann länger als der Stift des zweiten Kupplungskontakts ausgebildet sein. Demzufolge wird ein elektrisch leitender Kontakt der weiteren Kupplungskontakte in dem AUS-Zustand erreicht, während die zweiten Kupplungskontakte elektrisch und auch mechanisch außer Eingriff sind. Der Stift des weiteren Kupplungskontakts kann einen Isolierabschnitt aufweisen, der so ausgebildet ist, dass die weiteren Kupplungskontakte in dem AN-Zustand elektrisch außer Eingriff sind. Insbesondere kann ein Schaftabschnitt des als Stift ausgebildeten Kupplungskontakts mit einer Isolierschicht abgedeckt bzw. beschichtet sein, um den Isolierabschnitt zu bilden. Die Isolierschicht ist bevorzugt so gebildet, dass sie zu einem leitenden Abschnitt an der Spitze des Stifts im Wesentlichen plan ist. Alternativ oder zusätzlich kann die Buchse des weiteren Kupplungskontakts einen Isolierabschnitt aufweisen, der so ausgebildet ist, dass die dritten Kupplungskontakte in dem AN-Zustand elektrisch außer Eingriff sind. Insbesondere kann ein Bodenabschnitt der Buchse mit einer Isolierschicht abgedeckt sein, um den Isolierabschnitt zu bilden. Die Isolierschicht ist bevorzugt im Wesentlichen plan zu einem leitenden Abschnitt an einem Eingangsabschnitt der Buchse gebildet. Auf diese Weise wird eine elektrisch leitende Verbindung der weiteren Kupplungskontakte in dem AUS-Zustand und eine elektrisch nicht-leitende Verbindung in dem AN-Zustand erreicht.

Mit Vorteil können der weitere Kupplungskontakt und der erste Kupplungskontakt in der ersten Kupplungsvorrichtung über eine Drahtbrücke parallel miteinander verbunden sein. Demzufolge kann eine einfache Verbindung zwischen den beiden Kupplungskontakten erreicht werden. Die Drahtbrücke kann ggf. auch für vorhandene vierpolige erste Kupplungsvorrichtungen nachgerüstet werden.

Weiterhin kann die chirurgische Vorrichtung einen Erkennungswiderstand aufweisen, der mit der RFID-Antenne wenigstens in dem AUS-Zustand in Serie verbunden ist. Der Erkennungswiderstand kann dabei in der ersten Kupplungsvorrichtung oder in der zweiten Kupplungsvorrichtung angeordnet sein. Bevorzugt kann der Erkennungswiderstand in der ersten Kupplungsvorrichtung angeordnet sein und einen dem Anwendungsteil oder dem Elektromotor entsprechenden Widerstandswert aufweisen. Der Erkennungswiderstand kann beispielsweise einen Widerstandswert von 10 Ohm aufweisen. Demzufolge kann über den oben beschriebenen Pfad zum Versorgen der RFID-Antenne ein Serienwiderstand zwischen dem Erkennungswiderstand und dem Widerstand der RFID-Antenne vor und/oder während einer Anregung der RFID-Antenne durch die Steuereinheit erfasst werden, um das ordnungsgemäße Vorliegen des AUS-Zustands zu bestimmen. Dieses Vorgehen ist vor allem dann vorteilhaft, wenn die dritten Kupplungskontakte der ersten Kupplungsvorrichtung und der zweiten Kupplungsvorrichtung in dem AUS-Zustand elektrisch außer Eingriff sind und keine Messung des Serienwiderstands von Motorwicklungen möglich ist. Da der Serienwiderstand der Motorwicklungen um Bereich weniger Ohm liegt, ist das Vorsehen des Erkennungswiderstands aber auch dann vorteilhaft, wenn der Serienwiderstand der Motorwicklungen über die dritten Kupplungskontakte messbar ist.

Es hat sich als günstig erwiesen, wenn die erste Kupplungsvorrichtung eine Kupplungshülse aufweist, an der der RFID-Chip radial innenliegend angeordnet ist. Der RFID-Chip bzw. ein den RFID-Chip umgebendes Gussmaterial kann dabei bevorzugt im Wesentlichen plan zu einer Innenfläche der Kupplungshülse ausgebildet sein. Die erste Kupplungsvorrichtung kann als Kupplungszapfen ausgebildet sein, in dem wiederum ein Sackloch zum Bilden der Kupplungshülse geformt ist. Durch Anordnen des RFID-Chips, insbesondere des Glass-Tags, im Inneren der Kupplungshülse wird dieser zuverlässig vor einer mechanischen Beschädigung bewahrt. Zum Anordnen des RFID-Chips kann das aus der DE 10 2019 122 349 A1 bekannte Vorgehen verwendet werden. Weiterhin kann die Dicke einer Wandung des Kupplungszapfens, in dem die Kupplungshülse geformt ist, so gewählt werden, dass der RFID-Chip durch eine externe Vorrichtung, die eine RFID-Antenne aufweist und an den Kupplungszapfen im Bereich des RFID-Chips gehalten wird, auslesbar und/oder beschreibbar ist.

Vorteilhafterweise kann die zweite Kupplungsvorrichtung einen Kupplungszapfen aufweisen, an dem die RFID-Antenne radial außenliegend angeordnet ist. Die RFID-Antenne bzw. ein die RFID-Antenne umgebendes Gussmaterial kann bevorzugt im Wesentlichen plan zu einer Außenfläche des Kupplungszapfens ausgebildet sein. Die zweite Kupplungsvorrichtung kann als Kupplungshülse ausgebildet sein, in die wiederrum ein Kupplungszapfen hineinragt.

Mit Vorteil kann die RFID-Antenne in einer an der Außenfläche des Kupplungszapfens gebildeten Nut eingelassen, insbesondere eingegossen, sein. Die RFID-Antenne kann dabei bevorzugt ein Kupferdraht sein. Durch eine Führung der Nut an der Außenfläche des Kupplungszapfens kann demnach eine Form der RFID-Antenne festgelegt sein. Die RFID-Antenne kann insbesondere schleifenförmig, mäanderförmig, spiralförmig, spulenförmig oder wendelförmig ausgebildet sein. Demnach sind eine Richtwirkung und eine Reichweite der RFID-Antenne auf einfache Weise festlegbar.

Die RFID-Antenne kann bevorzugterweise als Schlaufe an einem distalen Ende des Kupplungszapfens angeordnet sein. Ein Abschnitt der Schlaufe kann in Umfangsrichtung des Kupplungszapfens, d.h. parallel zu einem stirnseitigen Ende des Kupplungszapfens, verlaufen. Demnach kann die RFID-Antenne auf einfache Weise gebildet sein und es wird sichergestellt, dass nur ein RFID-Chip in unmittelbarer Nähe der RFID-Antenne ausgelesen/beschrieben wird.

Die RFID-Antenne kann auch als metallische Beschichtung an einem distalen Ende des Kupplungszapfens angeordnet sein. Die RFID-Antenne kann somit auf einfache Weise gebildet werden. Die durch die metallische Beschichtung gebildete Antenne kann dabei auch in der an der Außenfläche des Kupplungszapfens gebildeten Nut eingelassen, insbesondere eingegossen, sein.

Mit Vorteil kann eine elektrische Schaltung vorgesehen sein, die so konfiguriert ist, dass sie die RFID-Antenne abstimmt. Die elektrische Schaltung kann dabei in der zweiten Kupplungsvorrichtung angeordnet sein. Demnach ist nur die RFID-Antenne im Bereich der zweiten Kupplungsvorrichtung abzustimmen. Alternativ kann die elektrische Schaltung in der Steuereinheit angeordnet sein. Folglich sind auch die Leitungen zum Versorgen der RFID-Antenne mit Energie abzustimmen. Durch die Abstimmung der RFID-Antenne mittels der elektrischen Schaltung wird eine zuverlässige Datenübertragung zwischen dem RFID-Chip und der Steuereinheit über die RFID-Antenne erreicht.

In der zweiten Kupplungsvorrichtung kann eine elektrische Schaltung angeordnet sein, die so konfiguriert ist, dass sie die von der RFID-Antenne empfangenen Daten verarbeitet und an die Steuereinheit überträgt und/oder die von der Steuereinheit empfangenen Daten verarbeitet und in den RFID-Chip schreibt. Die elektrische Schaltung kann dazu einen Mikrocomputer bzw. Mikrocontroller aufweisen. Die elektrische Schaltung kann die von der RFID-Antenne oder der Steuereinheit empfangenen Daten insbesondere von einer Form in eine andere Form und/oder wieder zurück wandeln. Bevorzugt kann die elektrische Schaltung die von der RFID-Antenne empfangenen Daten mittels der One-Wire-Technik an die Steuereinheit übertragen und/oder von dieser empfangen. Demzufolge wird eine zuverlässige bidirektionale Datenübertragung zwischen der elektrischen Schaltung und der Steuereinheit auch bei großen Kabellängen erreicht. Es ist anzumerken, dass die beiden beschriebenen elektrischen Schaltungen in einer elektrischen Schaltung integriert sein können.

### Kurzbeschreibung der Figuren

Nachfolgend wird die vorliegende Offenbarung unter Bezugnahme auf die Figuren beschrieben. Es zeigen:
Fig. 1 eine chirurgische Vorrichtung mit einem chirurgischen Anwendungsteil und einem elektrischen Versorgungskabel, die in einem AUS-Zustand miteinander gekoppelt sind,
Fig. 2 die chirurgische Vorrichtung mit dem chirurgischen Anwendungsteil und dem elektrischen Versorgungskabel, die in einem AN-Zustand miteinander gekoppelt sind,
Fig. 3 das chirurgische Anwendungsteil,
Fig. 4 eine erste Kupplungsvorrichtung des chirurgischen Anwendungsteils,
Fig. 5 eine Schnittansicht des chirurgischen Anwendungsteils,
Fig. 6 das elektrische Versorgungskabel,
Fig. 7 einen Kupplungszapfen einer zweiten Kupplungsvorrichtung des elektrischen Versorgungskabels,
Fig. 8 eine gedrehte Ansicht des Kupplungszapfens der zweiten Kupplungsvorrichtung,
Fig. 9 eine Schnittansicht der ersten und der zweiten Kupplungsvorrichtung, die in dem AUS-Zustand miteinander gekoppelt sind,
Fig. 10 eine detaillierte Schnittansicht der ersten und der zweiten Kupplungsvorrichtung, die in dem AUS-Zustand miteinander gekoppelt sind,
Fig. 11 eine weitere detaillierte Schnittansicht der ersten und der zweiten Kupplungsvorrichtung, die in dem AUS-Zustand miteinander gekoppelt sind,
Fig. 12 eine andere detaillierte Schnittansicht der ersten und der zweiten Kupplungsvorrichtung, die in dem AUS-Zustand miteinander gekoppelt sind,
Fig. 13 eine detaillierte Schnittansicht der ersten und der zweiten Kupplungsvorrichtung, die in dem AN-Zustand miteinander gekoppelt sind,
Fig. 14 eine weitere detaillierte Schnittansicht der ersten und der zweiten Kupplungsvorrichtung, die in dem AN-Zustand miteinander gekoppelt sind,
Fig. 15 ein schematisches Schaltkreisdiagramm in dem AUS-Zustand,
Fig. 16 ein schematisches Schaltkreisdiagramm in dem AUS-Zustand, in dem ein leitender Pfad zum Messen eines Serienwiderstands von zwei Motorwicklungen gezeigt ist,
Fig. 17 ein schematisches Schaltkreisdiagramm in dem AUS-Zustand, in dem ein leitender Pfad zum Versorgen einer RFID-Antenne mit Energie gezeigt ist,
Fig. 18 ein schematisches Schaltkreisdiagramm in dem AN-Zustand,
Fig. 19 ein schematisches Schaltkreisdiagramm in dem AUS-Zustand, in dem ein leitender Pfad zum Versorgen einer RFID-Antenne mit Energie gezeigt ist,
Fig. 20 ein schematisches Schaltkreisdiagramm in dem AN-Zustand,
Fig. 21 eine erste Kupplungsvorrichtung des chirurgischen Anwendungsteils,
Fig. 22 die erste Kupplungsvorrichtung aus Fig. 21, wobei ein inneres Elemente und ein Stirnseitenelement der Kupplungsvorrichtung nicht gezeigt sind,
Fig. 23 ein schematisches Schaltkreisdiagramm in dem AUS-Zustand, in dem ein leitender Pfad zum Versorgen einer RFID-Antenne mit Energie gezeigt ist,
Fig. 24 ein schematisches Schaltkreisdiagramm in dem AUS-Zustand, in dem ein leitender Pfad zum Messen eines Serienwiderstands von zwei Motorwicklungen gezeigt ist, und
Fig. 25 ein schematisches Schaltkreisdiagramm in dem AN-Zustand.

### Beschreibung von Ausführungsbeispielen

Nachstehend werden Ausführungsbeispiele der vorliegenden Offenbarung unter Bezugnahme auf die Figuren beschrieben. Es ist anzumerken, das gleichen oder funktional entsprechenden Teilen bzw. Abschnitten das gleiche Bezugszeichen zugewiesen ist.

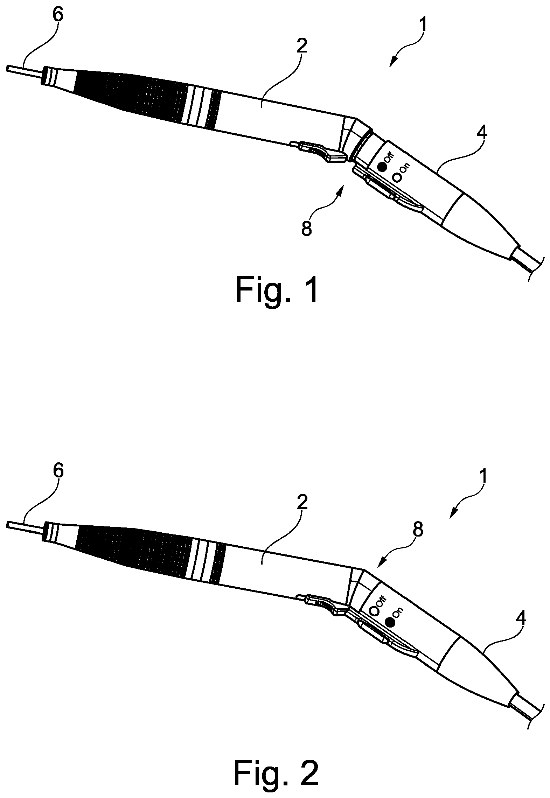

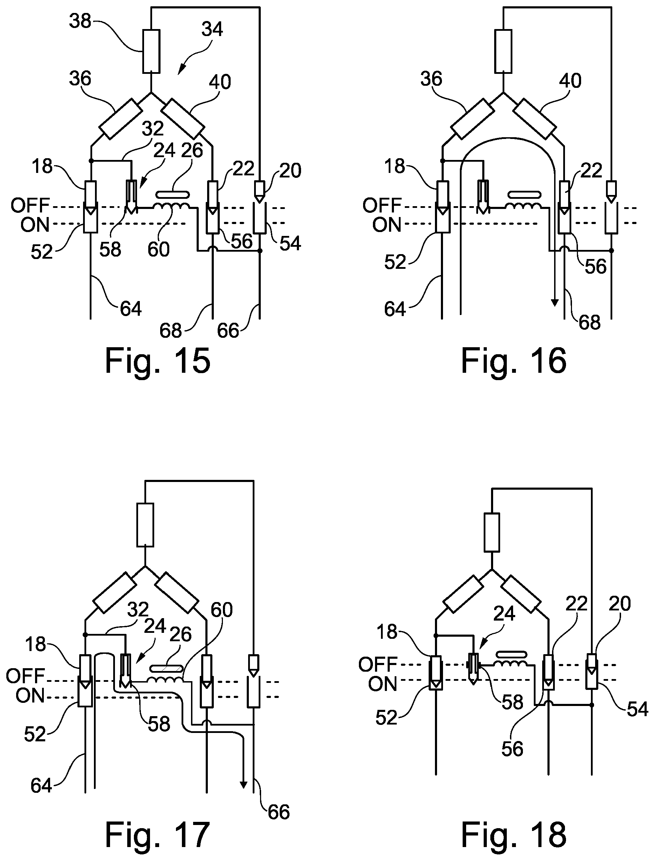

Die Figuren 1 und 2 zeigen eine chirurgische Vorrichtung 1, die ein chirurgisches Anwendungsteil 2, das z.B. eine Fräse, eine Säge usw. sein kann, und ein elektrisches Versorgungskabel 4 aufweist. An einem distalen Ende des Anwendungsteils 2 ist ein durch einen in dem Anwendungsteil 2 angeordneten Elektromotor 34 (siehe Fig. 15) antreibbares Werkzeug 6 angebracht. An einem proximalen Ende ist das Anwendungsteil 2 durch ein Kupplungssystem 8 (aus DE 10 2011 050 192 A1 bekannt) mit dem elektrischen Versorgungskabel 4 verbunden. Das Kupplungssystem 8 ist derart ausgebildet, dass es zwei Schaltzustände bzw. Kopplungszustände, nämlich einen AUS-Zustand (Fig. 1) und einen AN-Zustand (Fig. 2) ermöglicht.

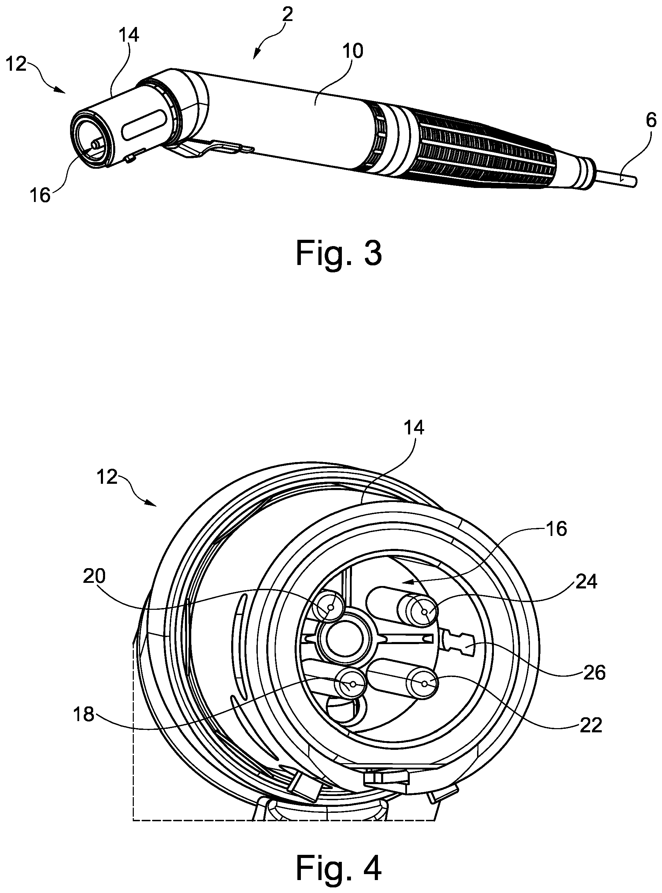

Fig. 3 zeigt das chirurgische Anwendungsteil 2 in einem Trennzustand, d.h. in einem Zustand, in dem das Kupplungssystem 8 außer Eingriff ist. Vorliegend ist das Anwendungsteil 2 als manuell haltbares bzw. betreibbares Anwendungsteil ausgebildet und weist daher einen Handgriff 10 auf. An einem proximalen Ende des Anwendungsteils 2 ist eine erste Kupplungsvorrichtung 12 des Kupplungssystems 8 angeordnet. Die Kupplungsvorrichtung 12 ist vorliegend als ein Kupplungszapfen 14 mit einer darin geformten Kupplungshülse 16 ausgebildet.

Fig. 4 zeigt eine detaillierte Ansicht der Kupplungsvorrichtung 12. In der Kupplungshülse 16 sind vier Kupplungskontakte, nämlich ein erster Kupplungskontakt 18, ein zweiter Kupplungskontakt 20, ein dritter Kupplungskontakt 22 und ein vierter oder weiterer Kupplungskontakt 24, angeordnet, die vorliegend als Stifte ausgebildet sind. An einer Innenfläche der Kupplungshülse 16 der ersten Kupplungsvorrichtung 12 ist ein RFID-Chip 26, der vorliegend als passiver Glas-Tag ausgebildet ist, im Wesentlichen plan zu der Innenfläche der Kupplungshülse 16 angeordnet.

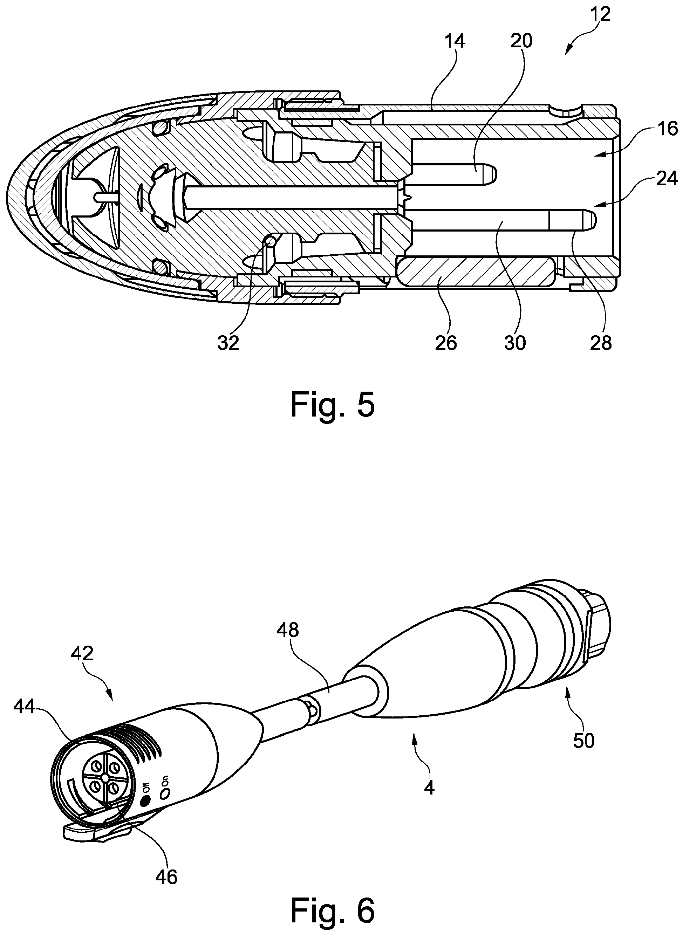

In Fig. 5 ist eine Schnittansicht der ersten Kupplungsvorrichtung 12 in einer Ebene des zweiten Kupplungskontakts 20 und des vierten Kupplungskontakts 24 zu sehen. Es ist zu erkennen, dass der zweite Kupplungskontakt 20 kürzer als der vierte Kupplungskontakt 24 ausgebildet ist. Außerdem weist der vierte Kupplungskontakt 24 zwei Abschnitte, nämlich einen leitenden Abschnitt 28 an seiner Spitze und einen Isolierabschnitt 30 an seinem Schaft auf. Darüber hinaus ist eine Drahtbrücke 32 zu erkennen.

Eine Verschaltung der Kupplungskontakte 18, 20, 22, 24 soll anhand von Fig. 15 erläutert werden. Der erste Kupplungskontakt 18 ist mit einer ersten Motorwicklung 36 verbunden. Der zweite Kupplungskontakt 20 ist mit der zweiten Motorwicklung 38 verbunden. Der dritte Kupplungskontakt 22 ist mit der dritten Motorwicklung 40 verbunden. Der vierte Kupplungskontakt 24 ist in dem Anwendungsteil 2 mit dem ersten Kupplungskontakt 18 über die Drahtbrücke 32 parallel verbunden.

Fig. 6 zeigt das elektrische Versorgungskabel 4, das an seinem distalen Ende eine zweite Kupplungsvorrichtung 42 aufweist, die als Kupplungshülse 44 mit einem darin ausgebildeten Kupplungszapfen 46 bereitgestellt ist. Die zweite Kupplungsvorrichtung 42 ist somit eingerichtet, um mit der ersten Kupplungsvorrichtung 12 mechanisch in Eingriff gebracht zu werden. Die zweite Kupplungsvorrichtung 34 ist über einen Kabelabschnitt 48, in dem eine der Anzahl der Motorwicklungen entsprechende Anzahl von Leitungen verläuft, mit einer Steuereinheit-Kupplungsvorrichtung 50, die ausgebildet ist, um mit einer Steuereinheit zum Betreiben des Elektromotors 34 verbindbar zu sein, verbunden.

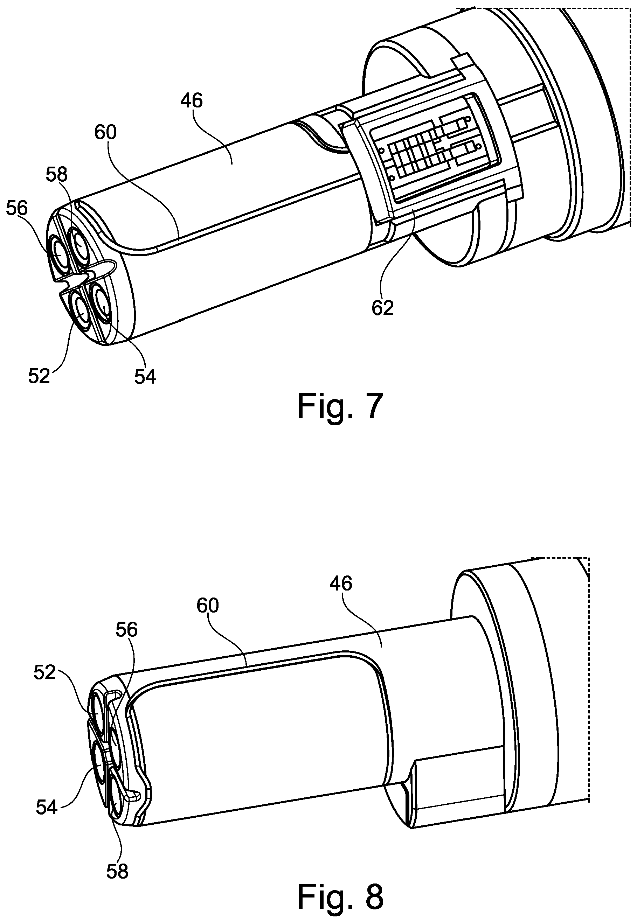

Die Figuren 7 und 8 zeigen eine detaillierte Ansicht des Kupplungszapfens 46 ohne die Kupplungshülse 44. In dem Kupplungszapfen 46 sind vier Kupplungskontakte, nämlich ein erster Kupplungskontakt 52, ein zweiter Kupplungskontakt 54, ein dritter Kupplungskontakt 56 und ein vierter oder weiterer Kupplungskontakt 58, eingelassen bzw. eingebracht. Vorliegend sind die Kupplungskontakte 52, 54, 56, 58 als Buchsen ausgebildet, die mit den als Stiften ausgebildeten Kupplungskontakten 18, 20, 22, 24 mechanisch in Eingriff gebracht werden können. An einer Außenfläche des Kupplungszapfen 46 ist eine RFID-Antenne 60 in einer dafür vorgesehenen Nut eingelassen. Vorliegend ist die RFID-Antenne 60 als eine Kupferdrahtschlaufe ausgebildet. Die Kupferdrahtschlaufe erstreckt sich an der Außenseite des Kupplungszapfens zunächst in axialer Richtung zu einer Stirnseite des Kupplungszapfens 46. Von dort verläuft die Schlaufe dann um einen vorgegebenen Winkel, z.B. 180°, entlang der Umfangsrichtung des Kupplungszapfens 46, d.h. parallel zu einer Stirnseite des Kupplungszapfens 46. Anschließend kehrt die Schlaufe über einen Abschnitt in axialer Richtung und einen Abschnitt entlang der Umfangsrichtung in die Nähe ihres Ausgangspunkts zurück. Demnach kann die RFID-Antenne 60 auf einfache Weise an der Außenfläche des Kupplungszapfens 46 angeordnet werden. Es ist anzumerken, dass diese Art der RFID- Antenne 60 beispielhaft ist und andere Antennenformen z.B. mäanderförmig, spiralförmig, spulenförmig oder wendelförmig, möglich sind. Alternativ kann auch eine andere Antennenart, z.B. gedruckte Schaltkreisplatine mit Leiterbahnen, flexible gedruckte Schaltkreisplatine mit Leiterbahnen, Kunststoff mit aktivierten Leiterbahnen, Zwei-Komponenten-Spritzguss mit Leiterbahnen oder Schlitzantenne, verwendet werden.

Die zweite Kupplungsvorrichtung 42 weist eine elektrische Schaltung 62 auf, die mit der RFID-Antenne 60 verbunden ist. Die elektrische Schaltung 62 kann so konfiguriert sein, dass sie die RFID-Antenne 60 abstimmt, um eine zuverlässige Datenübertragung zwischen dem RFID-Chip 26 und der Steuereinheit über die RFID-Antenne 60 zu ermöglichen. Alternativ oder zusätzlich kann die elektrische Schaltung 62 so konfiguriert sein, dass sie von der RFID-Antenne 60 empfangene Daten verarbeitet und an die Steuereinheit über Leitungen 64, 66 (siehe Fig. 15) des elektrischen Versorgungskabels 4, z.B. unter Verwendung der One-Wire-Technik, überträgt und/oder die von der Steuereinheit, z.B. unter Verwendung der One-Wire-Technik, empfangenen Daten verarbeitet und in den RFID-Chip 26 schreibt. Demzufolge kann das elektrische Versorgungskabel 4 eine größere Länge aufweisen, ohne dass eine Datenübertragung gestört wird.

Eine Verschaltung der Kupplungskontakte 52, 54, 56, 58 wird anhand von Fig. 15 beschrieben. Der erste Kupplungskontakt 52 ist mit einer ersten Leitung 64 verbunden. Der zweite Kupplungskontakt 54 ist mit einer zweiten Leitung 66 verbunden. Der dritte Kupplungskontakt 56 ist mit einer dritten Leitung 68 verbunden. Wie bereits beschrieben, verlaufen die Leitungen 64, 66, 68 in dem Kabelabschnitt 48 des elektrischen Versorgungskabels 4. Der vierte Kupplungskontakt 58 ist in der zweiten Kupplungsvorrichtung über die RFID-Antenne 60 mit der zweiten Leitung 66 verbunden.

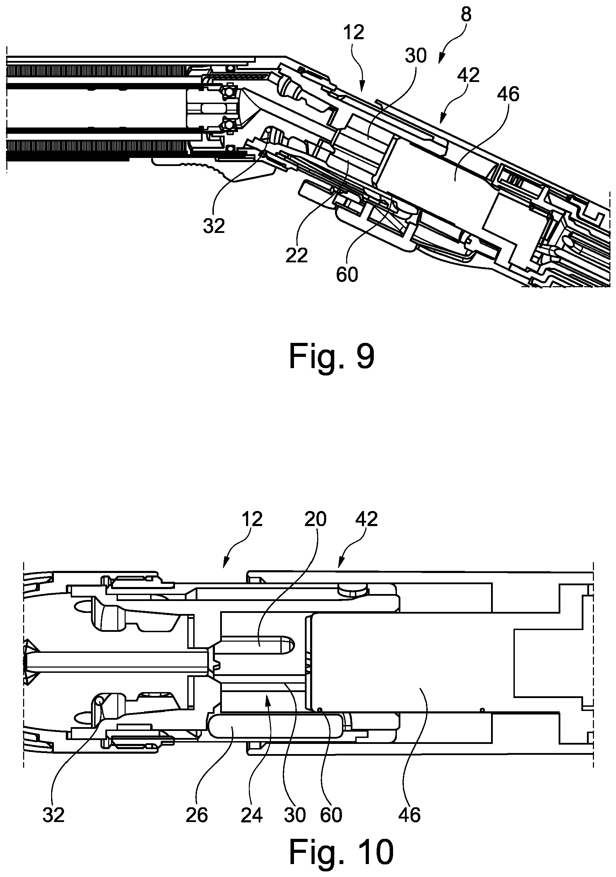

Nachfolgend werden Schaltzustände des Kupplungssystems 8 beschrieben. In Fig. 9 sind die erste Kupplungsvorrichtung 12 und die zweite Kupplungsvorrichtung 42 teilweise in Eingriff, um den AUS-Zustand zu erreichen. Es ist zu erkennen, dass der dritte Kupplungskontakt 22 und der vierte Kupplungskontakt 24, d.h. der leitende Abschnitt 28, teilweise in die dafür vorgesehenen Kupplungskontakte 56 bzw. 58 in dem Kupplungszapfen 46 hineinragen. Folglich ist der Isolierabschnitt 30 außerhalb des vierten Kupplungskontakts 58 angeordnet. Zudem ist die Drahtbrücke 32, die den ersten Kupplungskontakt 18 mit dem vierten Kupplungskontakt 24 verbindet, zu erkennen. Die Antenne 60 ist in der in Fig. 9 gezeigten Ansicht so angeordnet, dass der Schlaufenabschnitt entlang der Umfangsrichtung hinter der Bildebene angeordnet ist, um zu dem RFID-Chip 26 benachbart angeordnet zu sein.

Fig. 10 zeigt ebenfalls einen Fall, in dem das Kupplungssystem 8 in dem AUS-Zustand ist. Es ist zu erkennen, dass der zweite Kupplungskontakt 20 kürzer als die anderen Kupplungskontakte 18, 22, 24 ausgebildet ist, so dass er in dem AUS-Zustand nicht in den dafür vorgesehenen zweiten Kupplungskontakt 54 in dem Kupplungszapfen 46 hineinragt. Demzufolge ist eine Verbindung zwischen der zweiten Leitung 66 und der zweiten Motorwicklungen 38 zum Unterbinden eines Betriebs des Elektromotors 34 unterbrochen. In Fig. 10 ist zudem die benachbarte Anordnung der RFID-Antenne 60, insbesondere des entlang der Umfangsrichtung des Kupplungszapfens 46 verlaufenden Abschnitts, zu dem RFID-Chip 26 in dem AUS-Zustand zu erkennen.

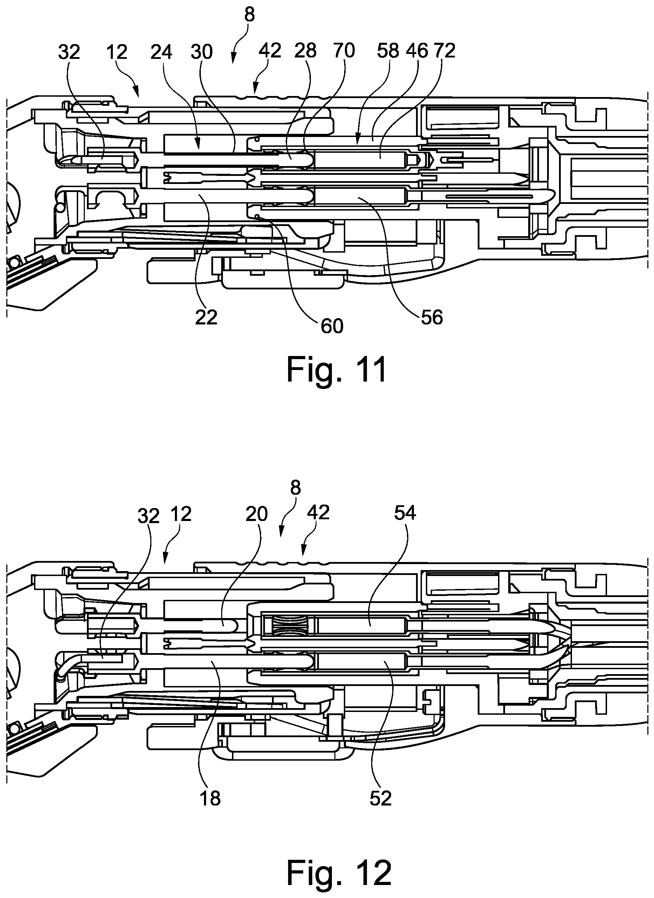

Fig. 11 zeigt eine Schnittansicht entlang einer Ebene der dritten und der vierten Kupplungskontakte 22, 24, 56, 58. Es ist zu erkennen, dass nur der leitende Abschnitt 28 des vierten Kupplungskontakt 24 in den dafür vorgesehenen vierten Kupplungskontakt 58 in dem Kupplungszapfen 46 hineinragt. Der vierte Kupplungskontakt 58 in dem Kupplungszapfen 46 weist an seinem Eingangsabschnitt einen leitenden Abschnitt 70 und an seinem Bodenabschnitt einen Isolierabschnitt 72 auf. In dem AUS-Zustand sind der leitende Abschnitt 28 des vierten Kupplungskontakts 24 und der leitende Abschnitt 70 des vierten Kupplungskontakts 58 sowohl mechanisch als auch elektrisch in Eingriff.

Fig. 12 zeigt eine Schnittansicht entlang einer Ebene der ersten und der zweiten Kupplungskontakte 18, 20, 52, 54. Es ist zu erkennen, dass der zweite Kupplungskontakt 20 und der dafür vorgesehene zweite Kupplungskontakt 54 in dem AUS-Zustand außer Eingriff sind.

In den Figuren 11 und 12 ist zudem der Verlauf der Drahtbrücke 32 von dem ersten Kupplungskontakt 18 zu dem vierten Kupplungskontakt 24, um diese parallel zu verbinden, zu erkennen. Vorliegend sind der erste Kupplungskontakt 18 und der vierte Kupplungskontakt 24 diagonal angeordnet. Die beiden Kupplungskontakte 18, 24 können aber auch nebeneinander angeordnet sein.

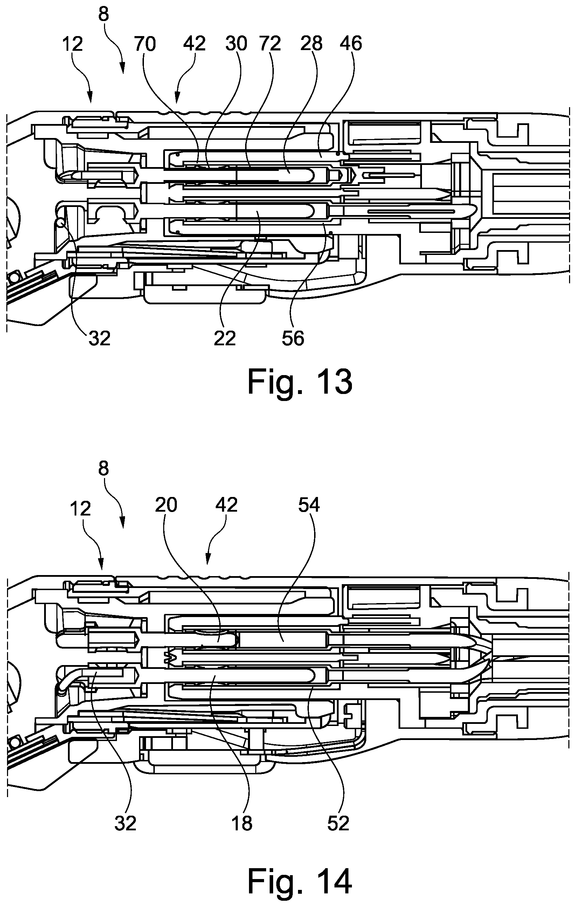

Fig. 13 zeigt eine Schnittansicht des Kupplungssystems 8 in dem AN-Zustand in der Ebene der dritten und der vierten Kupplungskontakte 22, 24, 56, 58. Es ist zu erkennen, dass die erste Kupplungsvorrichtung 12 vollständig in die zweite Kupplungsvorrichtung 42 eingreift, so dass der Kupplungszapfen 46 vollständig in die Kupplungshülse 16 eingeschoben ist. In dieser Konfiguration liegt der isolierende Abschnitt 30 des vierten Kupplungskontakts 24 gegenüber dem leitenden Abschnitt 70 des vierten Kupplungskontakts 58 und der leitende Abschnitt 28 des vierten Kupplungskontakts 24 liegt gegenüber dem Isolierabschnitt 72 des vierten Kupplungskontakts 58. Die vierten Kupplungskontakte 24, 58 sind demzufolge mechanisch in Eingriff, werden jedoch durch die oben beschriebene Anordnung der Isolierabschnitte 30, 72 und der leitenden Abschnitte 28, 70 elektrisch außer Eingriff, d.h. in einen nicht-leitenden Zustand, gebracht. Demzufolge wird eine elektrisch leitende Verbindung zwischen den vierten Kupplungskontakten 24, 58 zum Vermeiden eines Kurzschlusses der Motorwicklungen 36, 38 des Elektromotors 34 (siehe Fig. 15) unterbrochen.

Fig. 14 zeigt eine Schnittansicht des Kupplungssystems 8 in dem AN-Zustand in der Ebene der ersten und der zweiten Kupplungskontakte 18, 20, 52, 54. Es ist zu erkennen, dass nun auch der zweite Kupplungskontakt 20 und der dafür vorgesehene zweite Kupplungskontakt 54 sowohl elektrisch als auch mechanisch in Eingriff sind. Demzufolge wird eine leitende Verbindung zwischen der zweiten Leitung 66 und der zweiten Motorwicklung 38 erreicht. Folglich sind in dem AN-Zustand alle drei Motorwicklungen 36, 38, 40 mit den dafür vorgesehenen Leitungen 64, 66, 68 des elektrischen Versorgungskabels 4 elektrisch leitend verbunden, um einen Betrieb des Elektromotors 34 entsprechend der Ansteuerung durch die Steuereinheit zu ermöglichen.

Durch die oben beschriebene Konfiguration der Kupplungskontakte 18, 20, 22, 24 in der ersten Kupplungsvorrichtung 12 und der Kupplungskontakte 52, 54, 56, 58 in der zweiten Kupplungsvorrichtung 42 können nun folgende elektrische Verbindungen in dem AUS-Zustand und in dem AN-Zustand erreicht werden.

Wie in Fig. 16 durch einen Pfeil angedeutet, wird in dem AUS-Zustand eine leitende Verbindung über die erste Leitung 64, den ersten Kupplungskontakt 52, den ersten Kupplungskontakt 18, die erste Motorwicklung 36, die dritte Motorwicklung 40, den dritten Kupplungskontakt 22, den dritten Kupplungskontakt 56 und die dritte Leitung 68 erreicht. Demzufolge kann die Steuereinheit so konfiguriert sein, dass sie an die erste Leitung 64 und die dritte Leitung 68 ein Messsignal, insbesondere einen vorgegebenen Strom oder eine vorgegebene Spannung, anlegt, um einen Serienwiderstand der ersten Motorwicklung 36 und der dritten Motorwicklung 40 zu bestimmen. Demzufolge ist die Steuereinheit imstande, einen Typ des in dem Anwendungsteil 2 verbauten Elektromotors 34 anhand des gemessenen Serienwiderstands zu bestimmen.

Darüber hinaus wird, wie durch den Pfeil in Fig. 17 angezeigt, in dem AUS-Zustand eine leitende Verbindung über die erste Leitung 64, den ersten Kupplungskontakt 52, den ersten Kupplungskontakt 18, die Drahtbrücke 32, den dritten Kupplungskontakt 24, den dritten Kupplungskontakt 58, die RFID-Antenne 60 und die zweite Leitung 66 erhalten. Demzufolge wird die RFID-Antenne 60 mit Energie versorgt, um den benachbart angeordneten RFID-Chip 26 anzuregen und auszulesen oder Daten in den RFID-Chip 26 zu schreiben. Folglich kann eine Datenübertragung zwischen dem RFID-Chip 26 und der Steuereinheit erreicht werden. Beispielsweise können vor einer Verwendung des Anwendungsteils 2 Daten aus dem RFID-Chip 26 gelesen werden, so dass die Steuereinheit ermitteln kann, ob das Anwendungsteil 2 für die vorgesehene Verwendung geeignet ist. Nach einer Verwendung können die Daten in dem RFID-Chip 26 aktualisiert werden. Dieses Vorgehen ist beispielsweise auch vor und nach einer Wartung durchführbar.

Fig. 18 zeigt nun einen Fall, in dem das Kupplungssystem 8 in dem AN-Zustand ist. Folglich sind der vierte Kupplungskontakt 24 und der vierte Kupplungskontakt 58 elektrisch außer Eingriff und die Kupplungskontakte 18, 20, 22 der ersten Kupplungsvorrichtung 12 sind mit den dafür vorgesehenen Kupplungskontakten 52, 54, 56 der zweiten Kupplungsvorrichtung 42 elektrisch in Eingriff, um einen Betrieb des Elektromotors 34 entsprechend der Ansteuerung durch die Steuereinheit zu ermöglichen.

Obenstehend wurde ein Ausführungsbeispiel der vorliegenden Offenbarung beschreiben. Die vorliegende Offenbarung ist jedoch nicht auf das oben beschriebene Ausführungsbeispiel begrenzt und es kann folgende Modifikation angewendet werden.

Das Werkzeug 6 kann einen weiteren RFID-Chip aufweisen. In diesem Fall kann in dem Anwendungsteil 2 eine weitere RFID-Antenne benachbart zu dem weiteren RFID-Chip angeordnet sein, um den weiteren RFID-Chip auszulesen und/oder um Daten in den weiteren RFID-Chip zu schreiben. Die RFID-Antenne 60 und die weitere RFID-Antenne können dabei in Serie mit der Steuereinheit bzw. der elektrischen Schaltung 62 verbunden sein. Alternativ können die RFID-Antenne 60 und die weitere RFID-Antenne parallel in eigenen Schaltkreisen mit der Steuereinheit bzw. mit jeweiligen elektrischen Schaltungen 62 zum Abstimmen der RFID-Antennen verbunden sein. Durch Bereitstellen des weiteren RFID-Chips in dem Werkzeug 6 und der RFID-Antenne in dem Anwendungsteil 2 kann zusätzlich ein Auslesen/Schreiben von Daten über das Werkzeug 6 durch die Steuereinheit erfolgen. Demzufolge wird eine Transparenz in einem Service- bzw. Fehlerfall weiter erhöht.

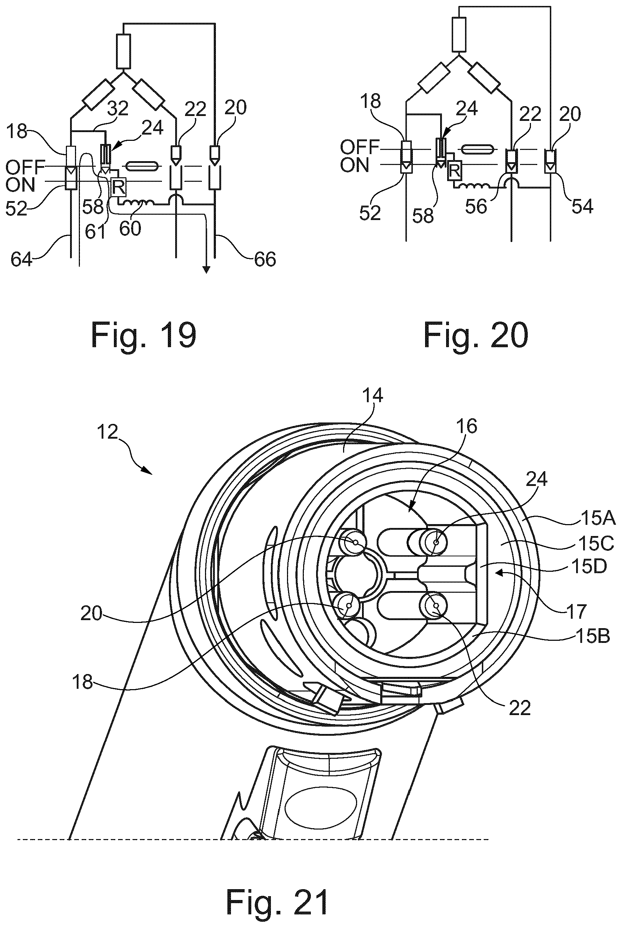

Obenstehend wurde ein Ausführungsbeispiel beschrieben, bei dem der dritte Kupplungskontakt 22 die gleiche Länge wie der erste und der vierte Kupplungskontakt 18, 24 aufweist. Gemäß einem weiteren Ausführungsbeispiel weist der dritte Kupplungskontakt 22 die gleiche Länge wie der zweite Kupplungskontakt 22 auf, d.h. ist kürzer als der erste und der vierte Kupplungskontakt 18, 24. Demzufolge ist der dritte Kupplungskontakt 22 in dem AUS-Zustand mit dem dritten Kupplungskontakt 56 elektrisch und mechanisch nicht in Eingriff. Ein entsprechendes Schaltkreisdiagramm ist in Fig. 19 gezeigt. Es ist zu erkennen, dass eine Anregung der RFID-Antenne 60 über die erste Leitung 64, den ersten Kupplungskontakt 52 in der zweiten Kupplungsvorrichtung 42, den ersten Kupplungskontakt 18 in der ersten Kupplungsvorrichtung 12, die Drahtbrücke 32, den vierten Kupplungskontakt 24 in der ersten Kupplungsvorrichtung 12, den vierten Kupplungskontakt 58 in der zweiten Kupplungsvorrichtung 42, einen Erkennungswiderstand 61, die RFID-Antenne 60 und die zweite Leitung 66 erfolgen kann.

Da jedoch die dritten Kupplungskontakte 22 und 56 bei diesem Ausführungsbeispiel elektrisch außer Eingriff sind, kann keine Messung des Serienwiderstands der der Motorwicklungen 36 und 40 erfolgen. Es ist daher vorteilhaft, wenn der Erkennungswiderstand 61 mit der RFID-Antenne 60 zumindest in dem AUS-Zustand in Serie verbunden ist. Die Steuereinheit kann dann vor und/oder während einer Anregung der RFID-Antenne 60 eine Widerstandsmessung durchführen, um das ordnungsgemäße Vorliegen des AUS-Zustands zu bestimmen. Beispielsweise kann der Erkennungswiderstand 61 einen Widerstandswert von 10 Ohm aufweisen. Ein Serienwiderstand über die RFID-Antenne 60 und den Erkennungswiderstand 61 ist dann beispielsweise ca. 12,7 Ohm. Es ist auch denkbar, dass der Erkennungswiderstand 61 in dem Anwendungsteil 2 angeordnet und entsprechend einem Typ des verwendeten Anwendungsteils 2 bzw. des verwendeten Elektromotors 34 codiert ist.

Darüber hinaus ist es auch denkbar, dass der Erkennungswiderstand 61 in dem in den Figuren 15 bis 19 gezeigten Ausführungsbeispiel vorgesehen ist. Es kann dann durch die Steuereinheit eine Vermessung des Serienwiderstands der Motorwicklungen 36, 40 und/oder eine Vermessung des Serienwiderstands über die RFID-Antenne 60 und den Erkennungswiderstand 61 erfolgen.

Fig. 20 zeigt ein Schaltkreisdiagramm bei Vorliegen des AN-Zustands. Es ist zu erkennen, dass die ersten bis dritten Kupplungskontakte 18, 20, 22, 52, 54, 56 nun elektrisch in Eingriff sind, während die vierten Kupplungskontakte 24, 58 elektrisch außer Eingriff sind. Demnach kann eine Ansteuerung des Elektromotors 34 durch die Steuereinheit erfolgen.

Fig. 21 zeigt ein weiteres Ausführungsbeispiel einer ersten Kupplungsvorrichtung 12 des Anwendungsteils 2. Die in Fig. 21 gezeigte Kupplungsvorrichtung 12 unterscheidet sich von der in Fig. 4 gezeigten Kupplungsvorrichtung 12 dadurch, dass ein Aufnahmeraum 17 gebildet ist, in dem der RFID-Chip 26 und die RFID-Antenne 60 benachbart zueinander angeordnet sind. Das bedeutet, sowohl der RFID-Chip 26 und die RFID-Antenne 60 sind in der ersten Kupplungsvorrichtung 12 angeordnet.

Wie in dem in Fig. 4 gezeigten Ausführungsbeispiel weist die erste Kupplungsvorrichtung 12 einen Kupplungszapfen 14 auf, in dem eine Kupplungshülse 16 gebildet ist, in dem die ersten bis vierten Kupplungskontakte 18, 20, 22, 24 aufgenommen sind. Zum Bilden des Kupplungszapfens 14 und der Kupplungshülse 16 weist die Kupplungsvorrichtung ein äußeres Element 15A, ein inneres Element 15B und ein Stirnseitenelement 15C auf. Das äußere Element 15A ist als zylindrischer Mantel gebildet und definiert eine äußere Form des Kupplungszapfens 14. Das innere Element 15B verläuft abschnittsweise parallel zu dem äußeren Element 15A und weist für den verbleibenden Umfang einen geraden Abschnitt 15D auf. Das innere Element 15B definiert somit eine Form der Kupplungshülse 16. Durch das Vorsehen des geraden Abschnitts 15D wird eine Wandstärke zwischen dem äußeren Element 15A und dem inneren Element 15B vergrößert, so dass der Aufnahmeraum 17 gebildet wird. Das Stirnseitenelement 15D verschließt eine Öffnung/einen Spalt zwischen dem äußeren Element 15A und dem inneren Element 15B zur Stirnseite der ersten Kupplungsvorrichtung 12 hin. Es ist selbsterklärend, dass eine Form des Kupplungszapfens 46 der zweiten Kupplungsvorrichtung 42 an die Form der Kupplungshülse 16 angepasst ist.

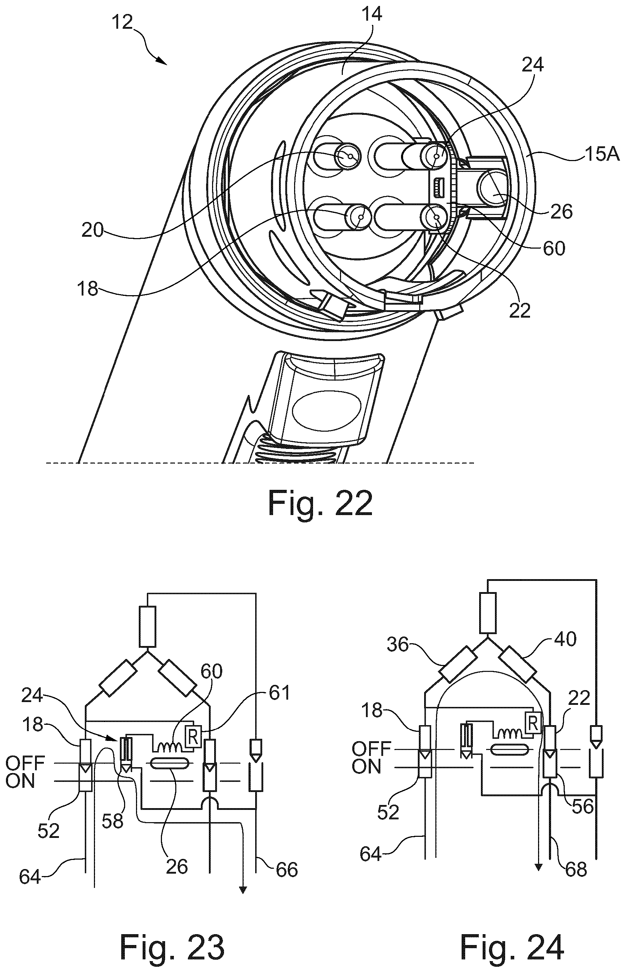

In Fig. 22 ist die erste Kupplungsvorrichtung 12 ohne das innere Element 15B und das Stirnseitenelement 15C gezeigt, um die Anordnung des RFID-Chips 16 und der RFID-Antenne 60 im Aufnahmeraum 17 darzustellen. Der RFID-Chip 26 ist radial außenliegend in dem Kupplungszapfen 16 angeordnet. Auf diese Weise kann der RFID-Chip 26 nicht nur durch die RFID-Antenne 16 ausgelesen/beschrieben werden. Es kann auch ein Auslesen/Beschreiben mittels einer externe Vorrichtung erfolgen, die hierfür eingerichtet und ausgebildet ist. Die externe Vorrichtung weist zu diesem Zweck unter anderem eine RFID-Antenne auf, um den RFID-Chip 26 anregen zu können. Die RFID-Antenne 60 ist benachbart und radial innenliegend zum RFID-Chip 26 angeordnet, so dass eine geringe Distanz zwischen dem RFID-Chip 26 und der RFID-Antenne 60 erhalten wird. Die RFID-Antenne 60 ist in dem in Fig. 22 gezeigten Ausführungsbeispiel als eine Schlitzantenne ausgebildet und entlang des geraden Abschnitts 15D angeordnet. Durch die Anordnung in der ersten Kupplungsvorrichtung 12 wird eine relative Anordnung zwischen dem RFID-Chip 26 und der RFID-Antenne 60 sichergestellt, so dass ein zuverlässiges Auslesen/Beschreiben des RFID-Chips 26 gewährleistet ist.

Fig. 23 zeigt ein entsprechendes Schaltkreisdiagramm in dem AUS-Zustand, in dem ein leitender Pfad zum Versorgen der RFID-Antenne 60 mit Energie durch einen Pfeil gezeigt ist. Eine Anregung der RIFD-Antenne 60 erfolgt demnach über die erste Leitung 64, den ersten Kupplungskontakt 52 in der zweiten Kupplungsvorrichtung 42, den ersten Kupplungskontakt 18 in der ersten Kupplungsvorrichtung 12, den Erkennungswiderstand 61, die RFID-Antennen 60, den vierten Kupplungskontakt 24 in der ersten Kupplungsvorrichtung 12, den vierten Kupplungskontakt 58 in der zweiten Kupplungsvorrichtung 42 und die zweite Leitung 66.

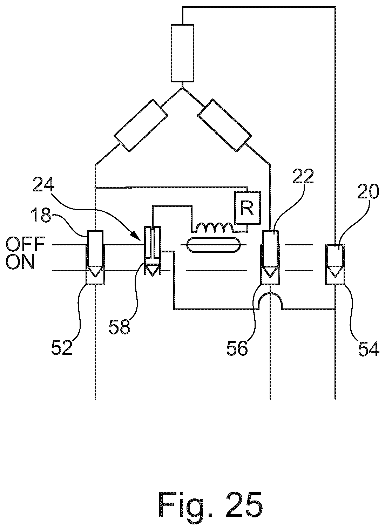

Fig. 24 zeigt die Vermessung eines Serienwiderstands der Motorwicklungen 36 und 40 in dem AUS-Zustand. Fig. 24 entspricht Fig. 16. Fig. 25 zeigt ein Schaltkreisdiagramm in dem AN-Zustand und entspricht Fig. 18.

Es ist anzumerken, dass in dem in den Figuren 23 bis 25 gezeigten Ausführungsbeispiel der Erkennungswiderstand 61 vorgesehen ist. Da die zweiten Kupplungskontakte 22 und 56 jedoch bereits in dem AUS-Zustand elektrisch und mechanisch in Eingriff sind und eine Vermessung des Serienwiderstands der Motorwicklungen 36 und 40 durch die Steuereinheit durchführbar ist, kann der Erkennungswiderstand 61 auch weggelassen werden.

Darüber hinaus ist es auch denkbar, dass in dem Ausführungsbeispiel, in dem der RFID-Chip 26 und die RFID-Antennen 60 in der ersten Kupplungsvorrichtung 12 angeordnet sind, der dritte Kupplungskontakt 22 dieselbe Länge wie der zweite Kupplungskontakt 20 aufweist, so dass die zweiten Kupplungskontakte 22, 52 in dem AUS-Zustand elektrisch und mechanisch nicht in Eingriff sind.

### Bezugszeichenliste

- 1: chirurgische Vorrichtung
- 2: chirurgisches Anwendungsteil
- 4: elektrisches Versorgungskabel
- 6: Werkzeug
- 8: Kupplungssystem
- 10: Handgriff
- 12: erste Kupplungsvorrichtung
- 14: Kupplungszapfen
- 15A: äußeres Element
- 15B: inneres Element
- 15C: Stirnseitenelement
- 15D: gerader Abschnitt
- 16: Kupplungshülse
- 17: Aufnahmeraum
- 18: erster Kupplungskontakt
- 20: zweiter Kupplungskontakt
- 22: dritter Kupplungskontakt
- 24: vierter Kupplungskontakt
- 26: RFID-Chip
- 28: leitender Abschnitt
- 30: Isolierabschnitt
- 32: Drahtbrücke
- 34: Elektromotor
- 36: erste Motorwicklung
- 38: zweite Motorwicklung
- 40: dritte Motorwicklung
- 42: zweite Kupplungsvorrichtung
- 44: Kupplungshülse
- 46: Kupplungszapfen
- 48: Kabelabschnitt
- 50: Steuereinheit-Kupplungsvorrichtung
- 52: erster Kupplungskontakt
- 54: zweiter Kupplungskontakt
- 56: dritter Kupplungskontakt
- 58: vierter Kupplungskontakt
- 60: RFID-Antenne
- 61: Erkennungswiderstand
- 62: elektrische Schaltung
- 64: erste Leitung
- 66: zweite Leitung
- 68: dritte Leitung
- 70: leitender Abschnitt
- 72: Isolierabschnitt

## Patentansprüche

1. Chirurgische Vorrichtung (1) aufweisend:
ein chirurgisches Anwendungsteil (2), das einen Elektromotor (34) mit Motorwicklungen (36, 38, 40) und an seinem einen Ende eine erste Kupplungsvorrichtung (12) aufweist,
ein elektrisches Versorgungskabel (4) mit einer einer Anzahl der Motorwicklungen (36, 38, 40) entsprechenden Anzahl von Leitungen, wobei das elektrische Versorgungskabel (4) an seinem einen Ende eine zweite Kupplungsvorrichtung (42), die zum Koppeln mit der ersten Kupplungsvorrichtung (12) eingerichtet ist, aufweist und an seinem zweiten Ende mit einer Steuereinheit zum Steuern des Elektromotors (34) verbunden oder verbindbar ist,
wobei die erste und die zweite Kupplungsvorrichtung (12, 42) so eingerichtet sind, dass sie in einen Trennzustand oder in einen AUS-Zustand, in dem erste Kupplungskontakte (18, 52) der ersten und der zweiten Kupplungsvorrichtung (12, 42) elektrisch in Eingriff sind und zweite Kupplungskontakte (20, 54) der ersten und der zweiten Kupplungsvorrichtung (12, 42) elektrisch außer Eingriff sind, oder in einen AN-Zustand, in dem die ersten Kupplungskontakte (18, 52) der ersten und der zweiten Kupplungsvorrichtung (12, 42) und die zweiten Kupplungskontakte (20, 54) der ersten und der zweiten Kupplungsvorrichtung (12, 42) elektrisch in Eingriff sind, bringbar sind,
einen in der ersten Kupplungsvorrichtung (12) angeordneten RFID-Chip (26), und
eine in der ersten Kupplungsvorrichtung (12) angeordnete und mit Energie versorgbare RFID-Antenne (60), die zu dem RFID-Chip (26) benachbart angeordnet ist und ausgebildet ist, den RFID-Chip (26) anzuregen, um Daten zwischen dem RFID-Chip (26) und der Steuereinheit, insbesondere bidirektional, zu übertragen,
wobei
die erste und die zweite Kupplungsvorrichtung (12, 42) jeweils einen weiteren Kupplungskontakt (24, 58) aufweisen, der in der ersten Kupplungsvorrichtung (12) über die RFID-Antenne (60) mit dem ersten Kupplungskontakt (18) parallel verbunden ist und in der zweiten Kupplungsvorrichtung (42) mit dem zweiten Kupplungskontakt (54) parallel verbunden ist, und
die weiteren Kupplungskontakte (24, 58) der ersten und der zweiten Kupplungsvorrichtung (12, 42) so eingerichtet sind, dass sie in dem AUS-Zustand elektrisch in Eingriff sind und in dem AN-Zustand elektrisch außer Eingriff sind.

2. Chirurgische Vorrichtung (1) gemäß Anspruch 1, **dadurch gekennzeichnet, dass**
die erste und die zweite Kupplungsvorrichtung (12, 42) dritte Kupplungskontakte (22, 56) aufweisen, die in dem AUS-Zustand und in dem AN-Zustand elektrisch in Eingriff sind.

3. Chirurgische Vorrichtung (1) gemäß Anspruch 1, **dadurch gekennzeichnet, dass**
die erste und die zweite Kupplungsvorrichtung (12, 42) dritte Kupplungskontakte (22, 56) aufweisen, die in dem AUS-Zustand elektrisch außer Eingriff und in dem AN-Zustand elektrisch in Eingriff sind.

4. Chirurgische Vorrichtung (1) gemäß einem der Ansprüche 1 bis 3, **dadurch gekennzeichnet, dass**
die Kupplungskontakte (18, 20, 22, 24) in der ersten Kupplungsvorrichtung (12) als Stifte und die Kupplungskontakte (52, 54, 56, 58) in der zweiten Kupplungsvorrichtung (42) als Buchsen ausgebildet sind,
in der ersten Kupplungsvorrichtung (12) der weitere Kupplungskontakt (24) länger als der zweite Kupplungskontakt (20) ausgebildet ist, und
die weiteren Kupplungskontakte (24, 58) Isolierabschnitte (30, 72) aufweisen, die so ausgebildet sind, dass die weiteren Kupplungskontakte (24, 58) in dem AN-Zustand elektrisch außer Eingriff sind.

5. Chirurgische Vorrichtung (1) gemäß einem der Ansprüche 1 bis 4, **dadurch gekennzeichnet, dass**
der weitere Kupplungskontakt (24) und der erste Kupplungskontakt (18) in der ersten Kupplungsvorrichtung (12) über eine Drahtbrücke (32) parallel miteinander verbunden sind.

6. Chirurgische Vorrichtung (1) gemäß einem der Ansprüche 1 bis 5, **dadurch gekennzeichnet, dass**
ein Erkennungswiderstand (61) mit der RFID-Antenne (60) wenigstens in dem AUS-Zustand in Serie verbunden ist.

7. Chirurgische Vorrichtung (1) gemäß einem der Ansprüche 1 bis 6, **dadurch gekennzeichnet, dass**
die erste Kupplungsvorrichtung (12) einen Kupplungszapfen (14) aufweist, in dem der RFID-Chip (26) radial außenliegend angeordnet ist.

8. Chirurgische Vorrichtung (1) gemäß Anspruch 7, **dadurch gekennzeichnet, dass**
die RFID-Antenne (60) in dem Kupplungszapfen (14) radial innenliegend zu dem RFID-Chip (26) angeordnet ist.

9. Chirurgische Vorrichtung (1) gemäß Anspruch 8, **dadurch gekennzeichnet, dass**
die RFID-Antenne (60) als Schlitzantenne ausgebildet ist.

10. Chirurgische Vorrichtung (1) aufweisend:
ein chirurgisches Anwendungsteil (2), das einen Elektromotor (34) mit Motorwicklungen (36, 38, 40) und an seinem einen Ende eine erste Kupplungsvorrichtung (12) aufweist,
ein elektrisches Versorgungskabel (4) mit einer einer Anzahl der Motorwicklungen (36, 38, 40) entsprechenden Anzahl von Leitungen, wobei das elektrische Versorgungskabel (4) an seinem einen Ende eine zweite Kupplungsvorrichtung (42), die zum Koppeln mit der ersten Kupplungsvorrichtung (12) eingerichtet ist, aufweist und an seinem zweiten Ende mit einer Steuereinheit zum Steuern des Elektromotors (34) verbunden oder verbindbar ist,
wobei die erste und die zweite Kupplungsvorrichtung (12, 42) so eingerichtet sind, dass sie in einen Trennzustand oder in einen AUS-Zustand, in dem erste Kupplungskontakte (18, 52) der ersten und der zweiten Kupplungsvorrichtung (12, 42) elektrisch in Eingriff sind und zweite Kupplungskontakte (20, 54) der ersten und der zweiten Kupplungsvorrichtung (12, 42) elektrisch außer Eingriff sind, oder in einen AN-Zustand, in dem die ersten Kupplungskontakte (18, 52) der ersten und der zweiten Kupplungsvorrichtung (12, 42) und die zweiten Kupplungskontakte (20, 54) der ersten und der zweiten Kupplungsvorrichtung (12, 42) elektrisch in Eingriff sind, bringbar sind,
einen in der ersten Kupplungsvorrichtung (12) angeordneten RFID-Chip (26), und
eine in der zweiten Kupplungsvorrichtung (42) angeordnete und mit Energie versorgbare RFID-Antenne (60), die zumindest in dem AUS-Zustand zu dem RFID-Chip (26) benachbart angeordnet ist und ausgebildet ist, den RFID-Chip (26) anzuregen, um Daten zwischen dem RFID-Chip (26) und der Steuereinheit, insbesondere bidirektional, zu übertragen,
wobei
die erste und die zweite Kupplungsvorrichtung (12, 42) jeweils einen weiteren Kupplungskontakt (24, 58) aufweisen, der in der ersten Kupplungsvorrichtung (12) mit dem ersten Kupplungskontakt (18) parallel verbunden ist und in der zweiten Kupplungsvorrichtung (42) über die RFID-Antenne (60) mit dem zweiten Kupplungskontakt (54) parallel verbunden ist, und
die weiteren Kupplungskontakte (24, 58) der ersten und der zweiten Kupplungsvorrichtung (12, 42) so eingerichtet sind, dass sie in dem AUS-Zustand elektrisch in Eingriff sind und in dem AN-Zustand elektrisch außer Eingriff sind.

11. Chirurgische Vorrichtung (1) gemäß Anspruch 10, **dadurch gekennzeichnet, dass**
die erste und die zweite Kupplungsvorrichtung (12, 42) dritte Kupplungskontakte (22, 56) aufweisen, die in dem AUS-Zustand und in dem AN-Zustand elektrisch in Eingriff sind.

12. Chirurgische Vorrichtung (1) gemäß Anspruch 10, **dadurch gekennzeichnet, dass**
die erste und die zweite Kupplungsvorrichtung (12, 42) dritte Kupplungskontakte (22, 56) aufweisen, die in dem AUS-Zustand elektrisch außer Eingriff und in dem AN-Zustand elektrisch in Eingriff sind.

13. Chirurgische Vorrichtung (1) gemäß einem der Ansprüche 10 bis 12, **dadurch gekennzeichnet, dass**
die Kupplungskontakte (18, 20, 22, 24) in der ersten Kupplungsvorrichtung (12) als Stifte und die Kupplungskontakte (52, 54, 56, 58) in der zweiten Kupplungsvorrichtung (42) als Buchsen ausgebildet sind,
in der ersten Kupplungsvorrichtung (12) der weitere Kupplungskontakt (24) länger als der zweite Kupplungskontakt (20) ausgebildet ist, und
die weiteren Kupplungskontakte (24, 58) Isolierabschnitte (30, 72) aufweisen, die so ausgebildet sind, dass die weiteren Kupplungskontakte (24, 58) in dem AN-Zustand elektrisch außer Eingriff sind.

14. Chirurgische Vorrichtung (1) gemäß einem der Ansprüche 10 bis 13, **dadurch gekennzeichnet, dass**
der weitere Kupplungskontakt (24) und der erste Kupplungskontakt (18) in der ersten Kupplungsvorrichtung (12) über eine Drahtbrücke (32) parallel miteinander verbunden sind.

15. Chirurgische Vorrichtung (1) gemäß einem der Ansprüche 10 bis 14, **dadurch gekennzeichnet, dass**
ein Erkennungswiderstand (61) mit der RFID-Antenne (60) wenigstens in dem AUS-Zustand in Serie verbunden ist.

## Claims

1. A surgical device (1), comprising:
a surgical application part (2) which includes an electric motor (34) with motor windings (36, 38, 40) and a first coupling device (12) at the one end thereof,
an electrical supply cable (4) with a number of lines corresponding to the number of motor windings (36, 38, 40), the electrical supply cable (4) including at the one end thereof a second coupling device (42) that is configured to be coupled to the first coupling device (12) and at the second end thereof being connected or connectable to a control unit for controlling the electric motor (34),
wherein the first and second coupling devices (12, 42) are configured so that they can be brought into a disconnecting state or into an OFF state in which the first coupling contacts (18, 52) of the first and second coupling devices (12, 42) are electrically in engagement and second coupling contacts (20, 54) of the first and second coupling devices (12, 42) are electrically out of engagement, or into an ON state in which the first coupling contacts (18, 52) of the first and second coupling devices (12, 42) and the second coupling contacts (20, 54) of the first and second coupling devices (12, 42) are electrically in engagement,
an RFID chip (26) arranged in the first coupling device (12), and
an RFID antenna (60) arranged in the first coupling device (12) and adapted to be supplied with energy which is arranged adjacent to the RFID chip (26) and is designed to excite the RFID chip (26) in order to transmit, specifically bidirectionally, data between the RFID chip (26) and the control unit,
wherein
each of the first and second coupling devices (12, 42) includes a further coupling contact (24, 58) which, in the first coupling device (12), is connected in parallel to the first coupling contact (18) via the RFID antenna (60) and, in the second coupling device (42), is connected in parallel to the second coupling contact (54), and
the further coupling contacts (24, 58) of the first and second coupling devices (12, 42) are configured so that, in the OFF state, they are electrically in engagement and, in the ON state, they are electrically out of engagement.

2. The surgical device (1) according to claim 1, **characterized in that**
the first and second coupling devices (12, 42) include third coupling contacts (22, 56) which are electrically in engagement in the OFF state and in the ON state.

3. The surgical device (1) according to claim 1, **characterized in that**
the first and second coupling devices (12, 42) include third coupling contacts (22, 56) which, in the OFF state, are electrically out of engagement and, in the ON state, are electrically in engagement.

4. The surgical device (1) according to any one of the claims 1 to 3,
**characterized in that**
the coupling contacts (18, 20, 22, 24) in the first coupling device (12) are in the form of pins and the coupling contacts (52, 54, 56, 58) in the second coupling device (42) are in the form of bushes,
in the first coupling device (12) the further coupling contact (24) is longer than the second coupling contact (20), and
the further coupling contacts (24, 58) include insulating sections (30, 72) which are designed so that, in the ON state, the further coupling contacts (24, 58) are electrically out of engagement.

5. The surgical device (1) according to any one of the claims 1 to 4,
**characterized in that**
the further coupling contact (24) and the first coupling contact (18) in the first coupling device (12) are connected in parallel to each other via a wire jumper (32).

6. The surgical device (1) according to any one of the claims 1 to 5,
**characterized in that**
a detection resistor (61) is connected in series to the RFID antenna (60) at least in the OFF state.

7. The surgical device (1) according to any one of the claims 1 to 6,
**characterized in that**
the first coupling device (12) includes a coupling plug (14) in which the RFID chip (26) is arranged radially on the outside.

8. The surgical device (1) according to claim 7, **characterized in that** the RFID antenna (60) is arranged in the coupling plug (14) radially on the inside relative to the RFID chip (26).

9. The surgical device (1) according to claim 8, **characterized in that** the RFID antenna (60) is a slot antenna.

10. A surgical device (1) comprising:
a surgical application part (2) that includes an electric motor (34) with motor windings (36, 38, 40) and a first coupling device (12) at the one end thereof,
an electrical supply cable (4) with a number of lines corresponding to the number of the motor windings (36, 38, 40), the electrical supply cable (4) at the one end thereof including a second coupling device (42) adapted to be coupled to the first coupling device (12), and at the second end thereof being connected or connectable to a control unit for controlling the electric motor (34),
wherein the first and second coupling devices (12, 42) are configured so that they can be brought into a disconnecting state or into an OFF state in which first coupling contacts (18, 52) of the first and second coupling devices (12, 42) are electrically in engagement and second coupling contacts (20, 54) of the first and second coupling devices (12, 42) are electrically out of engagement, or into an ON state in which the first coupling contacts (18, 52) of the first and second coupling devices (12, 42) and the second coupling contacts (20, 54) of the first and second coupling devices (12, 42) are electrically in engagement,
an RFID chip (26) arranged in the first coupling device (12), and
an RFID antenna (60) arranged in the second coupling device (42) and adapted to be supplied with energy which is arranged adjacent to the RFID chip (26) at least in the OFF state and is designed to excite the RFID chip (26) in order to transmit, specifically bidirectionally, data between the RFID chip (26) and the control unit,
wherein
each of the first and second coupling devices (12, 42) has a further coupling contact (24, 58) which, in the first coupling device (12), is connected in parallel to the first coupling contact (18) and, in the second coupling device (42), is connected in parallel to the second coupling contact (54) via the RFID antenna (60), and
the further coupling contacts (24, 58) of the first and second coupling devices (12, 42) are configured so that, in the OFF state, they are electrically in engagement and, in the ON state, they are electrically out of engagement.

11. The surgical device (1) according to claim 10, **characterized in that**
the first and second coupling devices (12, 42) include third coupling contacts (22, 56) which are electrically in engagement in the OFF state and in the ON state.

12. The surgical device (1) according to claim 10, **characterized in that**
the first and second coupling devices (12, 42) include third coupling contacts (22, 56) which, in the OFF state, are electrically out of engagement and, in the ON state, are electrically in engagement.

13. The surgical device (1) according to any one of the claims 10 to 12,
**characterized in that**
the coupling contacts (18, 20, 22, 24) in the first coupling device (12) are in the form of pins and the coupling contacts (52, 54, 56, 58) in the second coupling device (42) are in the form of bushes,
in the first coupling device (12), the further coupling contact (24) is longer than the second coupling contact (20), and
the further coupling contacts (24, 58) include insulating sections (30, 72) which are designed so that, in the ON state, the further coupling contacts (24, 58) are electrically out of engagement.

14. The surgical device (1) according to any one of the claims 10 to 13,
**characterized in that**
the further coupling contact (24) and the first coupling contact (18) in the first coupling device (12) are connected in parallel to each other via a wire jumper (32).

15. The surgical device (1) according to any one of the claims 10 to 14,
**characterized in that**
a detection resistor (61) is connected in series to the RFID antenna (60) at least in the OFF state.

## Revendications

1. Dispositif chirurgical (1) présentant :
une pièce d'application chirurgicale (2) qui présente un moteur électrique (34) avec des enroulements de moteur (36, 38, 40) et un premier dispositif d'accouplement (12) au niveau de l'une de ses extrémités,
un câble d'alimentation électrique (4) avec un nombre de lignes correspondant à un nombre des enroulements de moteur (36, 38, 40), dans lequel le câble d'alimentation électrique (4) présente, au niveau de l'une de ses extrémités, un deuxième dispositif d'accouplement (42) qui est configuré pour s'accoupler avec le premier dispositif d'accouplement (12), et est connecté ou peut être connecté au niveau de sa deuxième extrémité à un dispositif de commande pour commander le moteur électrique (34),
dans lequel les premier et deuxième dispositifs d'accouplement (12, 42) sont configurés de sorte qu'ils puissent être amenés dans un état de séparation ou dans un état ARRÊT dans lequel des premiers contacts d'accouplement (18, 52) des premier et deuxième dispositifs d'accouplement (12, 42) sont électriquement engagés et des deuxièmes contacts d'accouplement (20, 54) des premier et deuxième dispositifs d'accouplement (12, 42) sont électriquement désengagés, ou dans un état MARCHE dans lequel les premiers contacts d'accouplement (18, 52) des premier et deuxième dispositifs d'accouplement (12, 42) et les deuxièmes contacts d'accouplement (20, 54) des premier et deuxième dispositifs d'accouplement (12, 42) sont électriquement engagés,
une puce RFID (26) disposée dans le premier dispositif d'accouplement (12), et
une antenne RFID (60) disposée dans le premier dispositif d'accouplement (12) et pouvant être alimentée en énergie, qui est disposée de manière adjacente à la puce RFID (26) et est conçue pour exciter la puce RFID (26) afin de transmettre des données entre la puce RFID (26) et le dispositif de commande, en particulier de manière bidirectionnelle,
dans lequel
les premier et deuxième dispositifs d'accouplement (12, 42) présentent respectivement un contact d'accouplement supplémentaire (24, 58) qui, dans le premier dispositif d'accouplement (12), est connecté en parallèle au premier contact d'accouplement (18) via l'antenne RFID (60) et, dans le deuxième dispositif d'accouplement (42), connecté en parallèle au deuxième contact d'accouplement (54), et
les contacts d'accouplement supplémentaires (24, 58) des premier et deuxième dispositifs d'accouplement (12, 42) sont configurés de sorte qu'ils sont électriquement engagés à l'état ARRÊT et électriquement désengagés à l'état MARCHE.

2. Dispositif chirurgical (1) selon la revendication 1, **caractérisé en ce que** les premier et deuxième dispositifs d'accouplement (12, 42) présentent de troisièmes contacts d'accouplement (22, 56) qui sont électriquement engagés à l'état ARRÊT et à l'état MARCHE.

3. Dispositif chirurgical (1) selon la revendication 1, **caractérisé en ce que**
les premier et deuxième dispositifs d'accouplement (12, 42) présentent de troisièmes contacts d'accouplement (22, 56), qui sont électriquement désengagés à l'état ARRÊT et électriquement engagés à l'état MARCHE.

4. Dispositif chirurgical (1) selon l'une quelconque des revendications 1 à 3, **caractérisé en ce que**
les contacts d'accouplement (18, 20, 22, 24) du premier dispositif d'accouplement (12) sont conçus en tant que broches et les contacts d'accouplement (52, 54, 56, 58) dans le deuxième dispositif d'accouplement (42) sont conçus en tant que douilles,
dans le premier dispositif d'accouplement (12), le contact d'accouplement supplémentaire (24) est conçu plus long que le deuxième contact d'accouplement (20), et
les contacts d'accouplement supplémentaires (24, 58) présentent des sections d'isolation (30, 72), qui sont conçues de sorte que les contacts d'accouplement supplémentaires (24, 58) soient électriquement désengagés à l'état MARCHE.

5. Dispositif chirurgical (1) selon l'une quelconque des revendications 1 à 4, **caractérisé en ce que**
le contact d'accouplement supplémentaire (24) et le premier contact d'accouplement (18) dans le premier dispositif d'accouplement (12) sont connectés en parallèle l'un à l'autre via un fil de pontage (32).

6. Dispositif chirurgical (1) selon l'une quelconque des revendications 1 à 5, **caractérisé en ce que**
une résistance de détection (61) est connectée en série avec l'antenne RFID (60) au moins à l'état ARRÊT.

7. Dispositif chirurgical (1) selon l'une quelconque des revendications 1 à 6, **caractérisé en ce que**
le premier dispositif d'accouplement (12) présente un tenon d'accouplement (14) dans lequel la puce RFID (26) est disposée radialement à l'extérieur.

8. Dispositif chirurgical (1) selon la revendication 7, **caractérisé en ce que** l'antenne RFID (60) est disposée dans le tenon d'accouplement (14) radialement à l'intérieur par rapport à la puce RFID (26).

9. Dispositif chirurgical (1) selon la revendication 8, **caractérisé en ce que** l'antenne RFID (60) est conçue en tant qu'antenne à fente.

10. Dispositif chirurgical (1) présentant :
une pièce d'application chirurgicale (2) qui présente un moteur électrique (34) avec des enroulements de moteur (36, 38, 40) et un premier dispositif d'accouplement (12) au niveau de l'une de ses extrémités,
un câble d'alimentation électrique (4) avec un nombre de lignes correspondant à un nombre des enroulements de moteur (36, 38, 40), dans lequel le câble d'alimentation électrique (4) présente, au niveau de l'une de ses extrémités, un deuxième dispositif d'accouplement (42) qui est configuré pour s'accoupler avec le premier dispositif d'accouplement (12), et est connecté ou peut être connecté au niveau de sa deuxième extrémité à un dispositif de commande pour commander le moteur électrique (34),
dans lequel les premier et deuxième dispositifs d'accouplement (12, 42) sont configurés de sorte qu'ils puissent être amenés dans un état de séparation ou dans un état ARRÊT dans lequel des premiers contacts d'accouplement (18, 52) des premier et deuxième dispositifs d'accouplement (12, 42) sont électriquement engagés et des deuxièmes contacts d'accouplement (20, 54) des premier et deuxième dispositifs d'accouplement (12, 42) sont électriquement désengagés, ou dans un état MARCHE dans lequel les premiers contacts d'accouplement (18, 52) des premier et deuxième dispositifs d'accouplement (12, 42) et les deuxièmes contacts d'accouplement (20, 54) des premier et deuxième dispositifs d'accouplement (12, 42) sont électriquement engagés,
une puce RFID (26) disposée dans le premier dispositif d'accouplement (12), et
une antenne RFID (60) disposée dans le deuxième dispositif d'accouplement (42) et pouvant être alimentée en énergie, qui est disposée de manière adjacente à la puce RFID (26) au moins à l'état ARRÊT et est conçue pour exciter la puce RFID (26) afin de transmettre des données entre la puce RFID (26) et le dispositif de commande, en particulier de manière bidirectionnelle,
dans lequel
les premier et deuxième dispositifs d'accouplement (12, 42) présentent respectivement un contact d'accouplement supplémentaire (24, 58) qui, dans le premier dispositif d'accouplement (12), est connecté en parallèle au premier contact d'accouplement (18) et, dans le deuxième dispositif d'accouplement (42), connecté en parallèle au deuxième contact d'accouplement (54) via l'antenne RFID (60), et
les contacts d'accouplement supplémentaires (24, 58) des premier et deuxième dispositifs d'accouplement (12, 42) sont configurés de sorte qu'ils sont électriquement engagés à l'état ARRÊT et électriquement désengagés à l'état MARCHE.

11. Dispositif chirurgical (1) selon la revendication 10, **caractérisé en ce que**
les premier et deuxième dispositifs d'accouplement (12, 42) présentent de troisièmes contacts d'accouplement (22, 56), qui sont électriquement engagés à l'état ARRÊT et à l'état MARCHE.

12. Dispositif chirurgical (1) selon la revendication 10, **caractérisé en ce que**
les premier et deuxième dispositifs d'accouplement (12, 42) présentent de troisièmes contacts d'accouplement (22, 56), qui sont électriquement désengagés à l'état ARRÊT et électriquement engagés à l'état MARCHE.

13. Dispositif chirurgical (1) selon l'une quelconque des revendications 10 à 12, **caractérisé en ce que**
les contacts d'accouplement (18, 20, 22, 24) du premier dispositif d'accouplement (12) sont conçus en tant que broches et les contacts d'accouplement (52, 54, 56, 58) dans le deuxième dispositif d'accouplement (42) sont conçus en tant que douilles,
dans le premier dispositif d'accouplement (12), le contact d'accouplement supplémentaire (24) est conçu plus long que le deuxième contact d'accouplement (20), et
les contacts d'accouplement supplémentaires (24, 58) présentent des sections d'isolation (30, 72), qui sont conçues de sorte que les contacts d'accouplement supplémentaires (24, 58) soient électriquement désengagés à l'état MARCHE.

14. Dispositif chirurgical (1) selon l'une quelconque des revendications 10 à 13, **caractérisé en ce que**
le contact d'accouplement supplémentaire (24) et le premier contact d'accouplement (18) dans le premier dispositif d'accouplement (12) sont connectés en parallèle l'un à l'autre via un fil de pontage (32).

15. Dispositif chirurgical (1) selon l'une quelconque des revendications 10 à 14, **caractérisé en ce que**
une résistance de détection (61) est connectée en série avec l'antenne RFID (60) au moins à l'état ARRÊT.
